# EUROPÄISCHE PATENTSCHRIFT

(11) **EP 2 663 861 B1**
(45) Veröffentlichungstag und Bekanntmachung des Hinweises auf die Patenterteilung: **30.03.2016**
(21) Anmeldenummer: 12701438.9
(22) Anmeldetag: 10.01.2012
(51) Int. Cl.: G01N 33/50

(54) **VERFAHREN ZUR AUTOMATISIERTEN HERSTELLUNG EINER MOLEKÜLSCHICHT AUS AMPHIPHILEN MOLEKÜLEN UND VORRICHTUNG ZUM HERSTELLEN DIESER MOLEKÜLSCHICHT**
METHOD FOR THE AUTOMATED PRODUCTION CONSISTING OF A MOLECULAR LAYER OF AMPHIPHILIC MOLECULES AND A DEVICE FOR PRODUCING SAID MOLECULAR LAYER
PROCÉDÉ DE PRODUCTION AUTOMATISÉE D'UNE COUCHE MOLÉCULAIRE EN MOLÉCULES AMPHIPHILES ET DISPOSITIF POUR PRODUIRE CETTE COUCHE MOLÉCULAIRE

(30) Priorität: 10.01.2011 DE 102011008205
(43) Veröffentlichungstag der Anmeldung: 20.11.2013
(73) Patentinhaber: Albert-Ludwigs-Universität Freiburg, 79095 Freiburg (DE)
(72) Erfinder: BEHRENDS, Jan, 79252 Stegen (DE); BAAKEN, Gerhard, 79102 Freiburg (DE)
(74) Vertreter: Kirchner, Christian
(86) Internationale Anmeldenummer: PCT/EP2012/000081
(87) Internationale Veröffentlichungsnummer: WO 2012/095299

(56) Entgegenhaltungen:
- WO-A1-2006/068619
- CN-A- 101 704 685
- DE-A1-102010 022 929
- US-A1- 2009 167 288
- H SPEIJER ET AL: "Critical micelle concentrations and stirring are rate limiting in the loss of lipid mass during membrane degradation by phospholipase A2", BIOPHYSICAL JOURNAL, Bd. 70, Nr. 5, 1. Mai 1996 (1996-05-01), Seiten 2239-2247, XP55021500, ISSN: 0006-3495, DOI: 10.1016/S0006-3495(96)79789-8
- SPIE, PO BOX 10 BELLINGHAM WA 98227-0010 USA, Bd. 4199, Nr. 43, 2001, Seiten 86-93, XP040253127,

## Beschreibung

Die vorliegende Erfindung betrifft ein Verfahren zur automatisierten Herstellung einer Molekülschicht aus amphiphilen Molekülen, insbesondere einer Bilipidschicht, und eine Vorrichtung zum Herstellen dieser Molekülschicht.

Molekülschichten aus amphiphilen Molekülen, insbesondere Bilipidschichten (DoppelLipidschichten), werden z.B. in der zellulären Elektrophysiologie ebenso wie für bestimmte einzelmolekülanalytische Verfahren, die auf Nanoporen beruhen (molekularer Coulter-Counter), verwendet. Bei diesen Anwendungen setzt man insbesondere die Spannungsklemmtechnik ("voltage-clamp") ein, um eine präzise Messung von Flüssen geladener Teilchen (Ionen) zu erzielen. Die Messung des Ionenstroms erfolgt über eine dielektrische (isolierende) Trennschicht (Membran) zwischen zwei Elektrolytlösung enthaltenden Kompartimenten, die mindestens eine für Ionen durchlässige Pore bzw. einen lonenkanal enthält. Diese Membran kann eine Bilipidschicht sein, die der typische Grundbestandteil natürlicher biologischer (Zell-)Membranen ist und die deshalb als künstliches Modell einer natürlichen Zellmembran herangezogen werden kann. Für die Spannungsklemmtechnik ist es erforderlich, zwei Kompartimente, nämlich die elektrolytgefüllten Bereiche diesseits und jenseits der Membran, elektrisch zu kontaktieren. Zu diesem Zweck wird die Membran meist über der Apertur einer Trägersubstratoberfläche erzeugt, wobei die Apertur den oberen Rand einer Mikrokavität oder eines Mikrolochs in diesem Trägersubstrat bildet. Bringt man die Bilipidschicht über dieser Oberfläche auf, überspannt diese Molekülschicht die Apertur "freitragend" mittels der ihr eigenen Oberflächenspannung. Da diese Aperturen im optischen Mikroskop "schwarz" erscheinen, werden sie auch black Lipid Membranes" (BLM) genannt. Derartige Verfahren und Aufbauten für die Spannungsklemmmtechnik werden z.B. beschrieben in Baaken et al. ("Planar microelectrode-cavity array for high-resolution and parallel electrical recording of membrane ionic currents", Lab Chip, 2008, 8, 938-944), ferner auch in Baaken, Prucker, Behrends, Rühe 2005, European Cells and Materials 5, Suppl. 5, p. CS4; Baaken, Prucker, Sondermann, Behrends, Rühe 2007, Tissue Engineering 18, 889 oder auch in US 2009/0167288 A1. Ferner ist es auch von Interesse, Molekülschichten auf Apertur-freien Substraten zu erzeugen, um z.B. sogenannte "substratgestützte Membranen" ("supporte Membranes") zu erzeugen, für die sich bekanntermaßen wiederum eine ganze Reihe weiterer Anwendungen ergeben.

Die möglichst einfache und zuverlässige Erzeugung solcher Membranen auf Festkörpern beschäftigt die Forschung bereits seit langer Zeit. Zu den bekannten Verfahren zum Herstellen einer solchen Molekülschicht, insbesondere Bilipidschicht, auf einer Oberfläche gehören das Verfahren nach Langmuir-Blodgett/Langmuir-Schäfer, die Fusionierung von Lipidvesikeln, und die Streichmethode ("painting"). Auch das Dokument "Baaken et al." oder die US 2009/0167288 A1 fassen einige dieser Verfahren zusammen. Bei der Streichmethode werden in einem Lösungsmittel, z.B. Dekan, gelöste Lipide mithilfe eines "Pinsels" aus Draht mechanisch und von Hand über eine Oberfläche "gestrichen", die gegebenenfalls eine Apertur aufweist, so dass sich aufgrund der Selbstorganisation der amphiphilen Moleküle über der Oberfläche coplanar eine Bilipidschicht ausbildet. Diese von erfahrenem Fachpersonal durchzuführende Methode ist jedoch aufwändig und hinsichtlich der Anforderung nach geeigneter Reproduzierbarkeit der so erzeugten Membranen tendenziell nachteilig. Insbesondere besteht nach wie vor der Wunsch nach Verfahren zur zuverlässigen Herstellung solcher substratgestützter Molekülschichten und entsprechender Verbesserung der damit realisierbaren Sensoriken, um z.B. einen höheren Durchsatz an Messungen zu erreichen.

Es ist Aufgabe der vorliegenden Erfindung, ein Verfahren und eine Vorrichtung zur Herstellung einer Molekülschicht aus amphiphilen Molekülen, insbesondere einer Bilipidschicht, bereitzustellen, die insbesondere möglichst einfach und zuverlässig funktionieren.

Diese Aufgabe wird erfindungsgemäß gelöst durch das Verfahren gemäß Anspruch 1 und die Vorrichtung gemäß Anspruch 10. Bevorzugte Ausgestaltungen des Verfahrens bzw. der in diesem verwendeten Vorrichtung sind Gegenstände der Unteransprüche 2 bis 9.
Das erfindungsgemäße Verfahren zur automatisierten Herstellung einer Molekülschicht aus amphiphilen Molekülen, insbesondere Lipiden für eine Bilipidmembran, in einer Vorrichtung, die ein Trägersubstrat zum Tragen der Molekülschicht aufweist, ein Rotationselement, das über dem Trägersubstrat rotierbar ist, und eine Aktuatoreinrichtung, mittels der das Rotationselement automatisch rotierbar ist, wobei das Rotationselement eine Außenfläche aufweist, die zumindest abschnittsweise hydrophob ausgebildet ist, weist folgenden Verfahrensschritte auf:
   - Einbringen eines ersten Lösungsmittels, welches amphiphile Moleküle enthält, in einen Bereich oberhalb des Trägersubstrats;
   - Bewirken der automatischen Rotation des Rotationselements oberhalb des Trägersubstrats;
   - Bewegen des ersten Lösungsmittels zwischen dem Trägersubstrat und dem Rotationselement durch Wechselwirkung des rotierenden Rotationselement mit dem ersten Lösungsmittel, wodurch sich die Molekülschicht bildet.

Das erfindungsgemäße Verfahren weist insbesondere den Vorteil auf, dass die mechanisch bewirkte Herstellung der Molekülschicht durch eine nicht-manuelle, nämlich automatische, Bewegung des Rotationselements erfolgt, wodurch diese Herstellung einfach und zuverlässig ist. Das Verfahren verwendet insbesondere die erfindungsgemäße Vorrichtung.
Die erfindungsgemäße Vorrichtung zur automatisierten Herstellung einer Molekülschicht aus amphiphilen Molekülen auf einem Trägersubstrat, weist auf:
   ein Trägersubstrat zum Tragen der Molekülschicht,
   ein Rotationselement, das über dem Trägersubstrat rotierbar angeordnet ist, und
   eine Aktuatoreinrichtung, die das Rotationselement automatisch rotiert,
   wobei das Rotationselement eine Außenfläche aufweist, die zumindest abschnittsweise hydrophob ausgebildet ist,
   so dass ein Lösungsmittel, das die amphiphilen Moleküle enthält, bei einer Rotation mittels des Rotationselements auf dem Trägersubstrat bewegt wird, wodurch sich die Molekülschicht ausbreitet.

Bevorzugte Ausgestaltungen der erfindungsgemäßen Vorrichtung können nachfolgend auch der Beschreibung des erfindungsgemäßen Verfahrens entnommen werden und umgekehrt.

Das Trägersubstrat ist vorzugsweise zumindest abschnittsweise oder im wesentlichen vollständig planar ausgebildet. Das Trägersubstrat kann eine oder mehrere Mikrostrukturen aufweisen, also räumliche Hervorhebungen und/oder Vertiefungen mit kleinen Dimensionen, die z.B. wenigen Nanometer, einige wenige Mikrometer, einige wenige zehn Mikrometer oder einige wenige hundert Mikrometer betragen können. Solche Mikrostrukturen lassen sich z.B. durch bekannte optisch-litographische Verfahren erzeugen, bei denen mittels optischer Masken definierte Strukturen schichtweise auf einem Trägersubstrat aufgebracht werden und teilweise wieder entfernt werden.

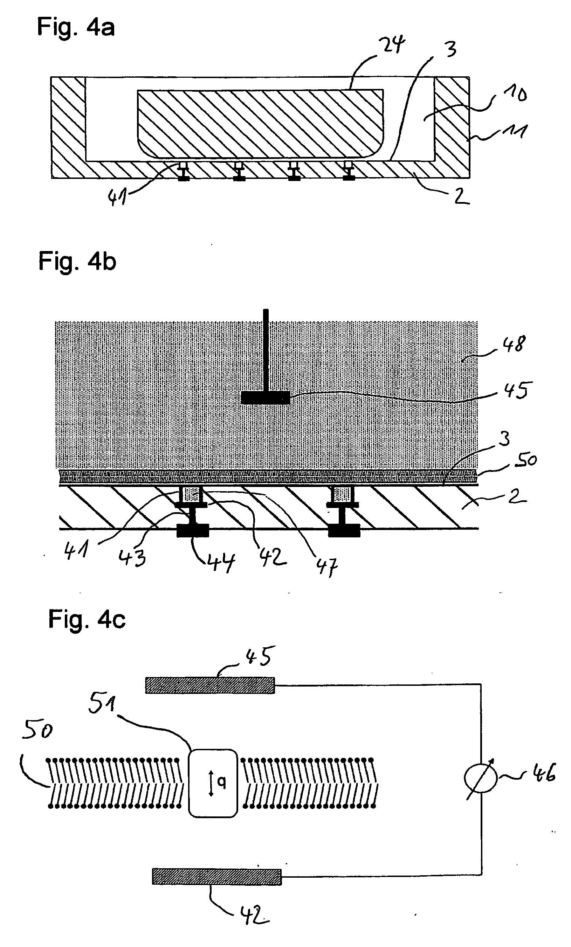

Das Trägersubstrat weist vorzugsweise eine Oberseite auf, die vorzugsweise zumindest abschnittsweise oder im wesentlichen vollständig planar ist. Die Oberseite weist vorzugsweise mindestens eine Apertur auf, vorzugsweise eine Anzahl N von Aperturen, wobei N jeweils vorzugsweise zwischen 2 und 2000, größer als 2000, vorzugsweise zwischen 2 und 400, zwischen 4 und 100, zwischen 4 und 50 oder zwischen 4 und 20 liegt. Mit einer Apertur kann z.B., wie eingangs beschrieben, eine freitragende Bilipidmembran (BLM) hergestellt werden. Die freitragende Molekülschicht trennt dann zwei Kompartimente voneinander, was Messanordnungen zur Realisierung einer Spannungsklemmtechnik ermöglicht. Dies wird später noch anhand Fig. 4 erläutert. Die Verwendung mehrere Aperturen hat den Vorteil, dass mehrere solcher Sensoriken parallel betrieben werden können, was einen höheren Messdurchsatz erlaubt.

Unter einer Apertur wird der offene Querschnitt verstanden, der sich z.B. durch eine Öffnung, z.B. eine Vertiefung oder ein Loch, in einer -insbesondere planaren-Oberfläche der Oberseite des Trägersubstrats ergibt. Die Form des Aperturumrisses ist vorzugsweise kreisförmig, ellipsoid, dreieckförmig, viereckförmig oder mehreckförmig. Der maximale, minimale oder durchschnittliche Durchmesser der einzelnen Apertur ist vorzugsweise kleiner als 1000 µm und liegt vorzugsweise zwischen 500 nm und 500 µm, zwischen 2 µm und 250 µm oder zwischen 5 µm und 150 µm. Bei solchen bevorzugten Aperturgrößen (Mikroaperturen) kann eine Molekülschicht über der Apertur erzeugt werden, was bei makroskopischen Aperturen mit Durchmessern von mehreren Millimetern in der Regel nicht möglich ist.

Solche Aperturen können durch selektives Entfernen einer lichtempfindlichen Schicht, z.B. Fotolack, die auf der Oberseite des Trägers angebracht ist, mittels optischer Litographie erzeugt werden, wie z.B. von banken et al" oder der US 2009/0167288 A1 beschrieben. Die Apertur kann aber auch den Rand eines Lochs bilden, das sich von der Oberseite bis zur Rückseite des Trägersubstrats erstreckt. Dies kann z.B. durch chemisches Ätzen oder durch Bestrahlung mit Laser- oder sonstigen hochenergetischen Strahlen erreicht werden.

Die Anordnung der Anzahl N von Aperturen entspricht vorzugsweise einem Array, vorzugsweise einem periodischen Gitter, in dem sich die Position der Aperturen bzw. der Apertur-Zentren durch einen oder wenige Gitterparameter beschreiben lässt. Die Anordnung in einem periodischen Gitter hat Vorteile beim Entwurf einer parallelisierten Sensorik, in der viele möglichst gleichartige Messstellen geschaffen werden sollen. Die Aperturen können aber auch in einem nicht-periodischen oder nicht vollständig periodischen Muster angeordnet sein.

Vozugsweise ist die Anordnung der Aperturen derart, dass alle Aperturen vollständig innerhalb der vom Rotationselement überstrichenen Fläche liegen, die von der Rotation eines einzelnen Rotationselements definiert ist. Auf diese Weise können mit einer einzigen Bewegung eines einzigen Rotationselements mehrere völlig gleichartige freitragende Molekülschichten hergestellt werden, was für die Reproduzierbarkeit und Zuverlässigkeit der damit realisierten Sensoriken von großem Vorteil ist. Vorzugsweise sind die Aperturen konzentrisch um einen Mittelpunkt angeordnet, und zwar vorzugsweise entlang mindestens einer Kreisbahn um diesen Mittelpunkt oder entlang mehrerer konzentrisch um diesen Mittelpunkt gelegener Kreisbahnen. Der Mittelpunkt ist vorzugsweise derart angeordnet, dass er dem Zentrum der Rotationsachse des Rotationselements entspricht. Der Effekt bei einer Anordnung entlang einer Kreisbahn ist, dass die Geschwindigkeit des Rotationselements dann in Höhe jeder Apertur einer Kreisbahn gleich ist. Durch das Rotationselement wird das erste Lösungsmittel bewegt, das die schichtbildenden amphiphilen Moleküle entält, wodurch sich die Molekülschicht ausbildet. Diese Bewegung erfolgt nun bei jeder Apertur mit gleicher Geschwindigkeit, wodurch gleichartige Membranen erzeugbar sind. Dies fördert die Reproduzierbarkeit der Ergebnisse bei Molekülschichtherstellung oder möglicher Sensorik. Eine solche Kreisbahn weist vorzugsweise einen Radius von mindestens fünf oder zehn Aperturdurchmessern bis zu Millimetern auf. Dies bietet Vorteile, wenn sich das erste Lösungsmittel getrieben durch das Rotationselement und seine eigene trägheitsbedingte Zentrifugalkraft aus dem Zentrum heraus radial nach außen ausbreitet. Durch diese Einflüsse wird die Bewegung des ersten Lösungsmitttels radial außerhalb des Bereichs um den Mittelpunkt der Rotation normalisiert". Jede Apertur erfährt so die gleiche Herstellungsprozedur. Aus diesem Grund ist auch bevorzugt, dass sich zumindest innerhalb einer inneren Kreisbahn um den Mittelpunkt keine Apertur befindet (azentrische Anordnung der Apertur/Aperturen). Es ist aber dennoch möglich und bevorzugt, dass eine oder mehrere Aperturen innerhalb dieser inneren Kreisbahn um den Mittelpunkt angeordnet sind, insbesondere zentrisch angeordnet sind.

Das Trägersubstrat ist vorzugsweise aus Glas hergestellt oder weist Glas auf. Es kann aber auch aus einem Halbleitermaterial bestehen oder dieses zumindest aufweisen, z.B. Si/SiO2. Andere Materialien sind ebenfalls möglich. Die Oberseite des Trägersubstrats weist vorzugsweise eine Beschichtung auf. Diese ist vorzugsweise hydrophob, kann aber auch weniger hydrophob sein und kann auch hydrophil sein. Der Vorteil einer hydrophoben Oberseite ist, dass sich viele Arten von Bilipidschichten auf solchen Oberflächen besonders zuverlässig ausbilden.

Unter einer "hydrophoben Grenzschicht, insbesondere nun bereits in Bezug auf die Gestaltung des Rotationselements, wird im Rahmen der vorliegenden Erfindung eine Schicht verstanden, auf der ein Wassertropfen einen Kontaktwinkel von mindestens 70° aufweist, vorzugsweise zwischen 80° und 130° oder zwischen 90° und 120°. Insofern ist die vorliegende Definition des Begriffs "hydrophob" breiter als allgemein in der Literatur üblich, wo der Begriff meist Kontaktwinkel von größer als 90° bezeichnet. Solche Kontaktwinkel (Innenwinkel des Wassertropfens) lassen sich leicht mittels im Handel erhältlicher Kontaktwinkelmessgeräte oder durch Auswertung lichtmikroskopischer Querschnittsbilder der Tropfen ermitteln (z.B. bei Raumtemperatur und unter Standardbedingungen).

Diese Beschichtung des Trägersubstrats, die vorzugsweise dessen Oberseite bildet und vorzugsweise hydrophob ist, besteht vorzugsweise aus einer lichtempfindlichen Schicht, insbesondere Fotolack, oder weist diese auf. Dieser Fotolack ist vorzugsweise SU8, dessen getrocknete Schichten hydrophob sind. SU-8 ist ein kommerziell erhältlicher Fotolack der Firma Microchem Corp., USA, und gehört zu der Gruppe der Negativ-Resiste. Wie die meisten Resiste besteht SU-8 aus den drei Bestandteilen Grundharz, Lösungsmittel und fotoempfindlicher Komponente. Diese eignen sich besonders zur Bereitstellung des Trägersubstrats der erfindungsgemäßen Vorrichtung, da sich insbesondere viele Bilipidschichten auf diesen Resisten besonders zuverlässig ausbilden und da diese photolitographisch bearbeitbar sind, um z.B. Mikrostrukturen herzustellen, und chemisch relativ inert sind.

Die Beschichtung des Trägersubstrats kann ferner vorzugsweise Polytetrafluorethylen (PTFE) aufweisen oder aus diesem Material bestehen. Der Vorteil ist, dass solche Schichten chemisch besonders inert sind, wodurch sie sich besonders zur Anwendung in korrosiven Umgebungen eignen (z.B. physiologischen Elektrolyten- physiologischen Salzlösungen).

Das Trägersubstrat ist vorzugsweise integraler Bestandteil der erfindungsgemäßen Vorrichtung. Das ist z.B. vorteilhaft, wenn dieselbe Vorrichtung, z.B. nach Reinigung, für wechselnde Molekülschichten verwendet werden soll.

Das Trägersubstrat kann aber vorzugsweise auch als separates, von der Vorrichtung lösbares Modulteil vorgesehen sein, so dass mittels der erfindungsgemäßen Vorrichtung eine Molekülschicht auf dem Trägersubstrat hergestellt wird, und danach das Trägersubstrat mit dieser Molekülschicht bezüglich der Vorrichtung anders angeordnet wird, insbesondere dieser Vorrichtung entnommen wird. Alternativ kann auch das Trägersubstrat fest angeordnet sein, z.B. fest über einer Messstation angeordnet sein, und die erfindungsgemäße Vorrichtung kann transportierbar, also insbesondere gegenüber dem Trägersubstrat und/oder der Messstation beweglich angeordnet sein. Wenn also das Trägersubstrat und die erfindungsgemäße Vorrichtung (d.h. deren Rotationselement und/oder deren Aktuatoreinrichtung), gegeneinander versetzbar angeordnet sind, ergibt sich der Vorteil, dass mittels einer einzigen Vorrichtung auf mehreren Trägersubstraten zahlreiche Molekülschichten erzeugt werden können. Dies ist insbesondere für einen automatischen Ablauf und einen hohen Durchsatz bei Herstellung und Messung solcher Molekülschichten von Vorteil.

Es ist auch möglich und bevorzugt, dass in einer einzigen Vorrichtung mehrere Trägersubstrate angeordnet sind, insbesondere coplanar angeordnet sind.

Die Vorrichtung weist vorzugsweise eine Halteeinrichtung auf, mittels der das Trägersubstrat gehalten wird, vorzugsweise lösbar gehalten wird, insbesondere mittels einer Klemmeinrichtung.

Vorzugsweise weist ein Trägersubstrat mindestens eine Mikrokavität auf, oder vorzugsweise ein Array von Mikrokavitäten auf, wobei eine oder jede Mikrokavität nach oben offen ist und in einer der genannten Aperturen (Mikroaperturen) in der Oberseite des Trägersubstrats mündet. Die herzustellende Molekülschicht bildet sich dann so, dass sie die mindestens eine Apertur oder mehrere Aperturen überdeckt. Eine Mikrokavität ist einer Vertiefung in der Oberseite, deren Tiefe dieselbe Größe wie ein möglicher genannter Aperturdurchmesser haben kann, oder tiefer sein kann. Jeder Querschnitt durch die Vertiefung weist vorzugsweise denselben Querschnitt auf wie die Apertur, welche die Mikrokavität nach oben öffnet. Die Mikrokavität kann insbesondere zylinderartig oder quaderartig sein. Sie kann aber auch hohlkegel(stumpf)förmig sein oder eine andere Form mit veränderlichem Querschnitt aufweisen. Eine Mikrokavität kann als Kompartiment dienen, in dem Elektrolyt zum Kontaktieren der Unterseite einer auf der Apertur angeordneten Molekülschicht angeordnet wird. Am Boden der Mikrokavität kann eine Elektrode angeordnet sein, mittels der der Elektrolyt in diesem Kompartiment elektrisch kontaktiert wird (entsprechend der Anordnung in Fig. 4a-c). Mithilfe einer Gegenelektrode im Elektrolyt des ersten Kompartiments oberhalb der trennenden Membran kann eine Spannungsklemmtechnik-Messanordnung realisiert werden.

Ein Trägersubstrat kann ferner an seiner Oberseite mindestens einen Sockelabschnitt oder mehrere Sockelabschnitte aufweisen, die vorzugsweise das darüber rotierende Rotationselement auf Distanz halten, indem das Rotationselement zwar die Sockelabschnitte berühren kann, nicht aber den Bereich der Oberseite, in dem sich die Molekülschicht ausbilden soll. Ein Sockelabschnitt kann sich z.B. nockenartig aus der Oberseite des Trägersubstrats erheben. Die Höhe dieser Sockelabschnitte kann z.B. 100 nm bis 1000 µm betragen, oder 500 nm bis 500 µm oder 1 µm bis 250 µm oder 50 µm bis 250 µm. Auf diese Weise kann die Qualität und Uniformität der entstehenden Membran weiter verbessert werden, da sich Unebenheiten auf der Kontaktseite des Rotationselements nicht direkt auf die Membran auswirken (weniger "Kratzen"). Ein Sockelabschnitt kann beliebige Form aufweisen. Er hat selber eine vorzugsweise planare Oberfläche, was den rotierenden Kontakt begünstigt. Diese Oberfläche weist vorzugsweise PTFE auf. Ein Sockelabschnitt kann auch als Führungselement ausgebildet sein, welches das Rotationselement während der Rotationsbewegung führt, und kann z.B. als kreisringartige Erhebung auf der Oberseite des Trägersubstrats ausgebildet sein. Auf diese Weise kann mit noch höherer Präzision sichergestellt werden, dass nur solche ausgewählten Bereiche der Kontaktseite des Rotationselementes oberhalb eines Zielbereichs rotieren, welche eine besondere Ausgestaltung (z.B. Beschichtung) aufweisen können, so dass in diesem Zielbereich die Molekülschicht in besonders kontrollierter Weise hergestellt werden soll. Ferner kann auch die Kontaktseite des Rotationselements mindestens einen Sockelabschnitt oder mehrere Sockelabschnitte aufweisen, welche ggf. die genannten Funktionen übernehmen.

Die Vorrichtung weist ferner vorzugsweise mindestens einen Wandabschnitt auf, der oberhalb des Trägersubstrats angeordnet ist und mit dem Trägersubstrat eine Kammer definiert, in der ein Flüssigkeitsvolumen von einigen Mikolitern und vorzugsweise bis zu einem Milliliter anordenbar ist. Somit kann die Vorrichtung einen Kammerabschnitt zur Aufnahme eines solchen Flüssigkeitsvolumens, insbesondere eines ersten und eines zweiten Lösungsmittels (Elektrolyts) aufweisen.

Das Rotationselement ist vorzugsweise stabartig ausgebildet. Mittels einer solchen materialsparenden Form kann eine Fläche überstrichen werden, die vorzugsweise (L/2)^2*pi entspricht, wobei U2 der Radius der durch die Rotation der Kontaktseite des Rotationselements überstrichenen- Kreisfläche ist. Im Bereich dieser Kreisfläche kontaktiert das Rotationselement das Trägersubstrat bzw. die dort angeordnete Schicht des ersten Lösungsmittels und erzeugt so im Bereich dieser Kreisfläche die Molekülschicht. Falls die Rotation des Rotationselements aber nicht mindestens einmal 360° beträgt, was ebenfalls möglich ist, so ist diese rotierend überstrichene Fläche kleiner als diese Kreisfläche und entspricht einem oder zwei Kreissegmenten.

Das Rotationselement kann so ausgestaltet sein, dass der Wert L für den Durchmesser der überstrichenen Kreisfläche zwischen 1 mm und 100 mm beträgt, vorzugsweise zwischen 3 mm und 15 mm, oder zwischen 5 mm und 10 mm. Das Rotationselement kann beliebige Form aufweisen, solange eine Kontaktseite existiert, welche das erste Lösungsmittel auf einem geeignet geformten Trägersubstrat effektiv bewegen und verteilen kann. Das Rotationselement ist vorzugsweise stab- oder quaderförmig, weist diese Länge L auf und ferner vorzugsweise einen durchschnittlichen oder maximalen Durchmesser D, wobei vorzugsweise D zwischen 0,5 mm und 25 mm oder 1 mm und 10 mm liegt, oder vorzugsweise zwischen 2 mm und 5 mm.

Vorzugsweise ist das Rotationselement ein flaches Element, d.h. seine durchschnittliche Höhe ist geringer, vorzugsweise mehr als zweimal geringer, als seine durchschnittliche (oder maximale) Breite und/oder durchschnittliche (oder maximale) Länge. Das Rotationselement kann ferner auch scheibenförmig, sternkörperartig (z.B. mit drei, vier, fünf, sechs, oder mehr Zackenelementen), radartig (Achsenelement mit Reifenelement(en) und Speichenelementen) oder anders ausgebildet sein.

Das Rotationselement weist vorzugsweise eine Kontaktseite auf, die dem Trägersubstrat zugewandt angeordnet (oder so anordenbar) ist. Die Kontaktseite dient dazu, das Trägersubstrat und/oder die auf dem Trägersubstrat angeordnete Schicht aus dem ersten Lösungsmittel, welches die amphiphilen Moleküle enthält, zu kontaktieren. Mittels dieses Kontakts wird das erste Lösungsmittel oberhalb des Trägersubstrats bewegt, wodurch sich diese Molekülschicht bildet. Die Kontaktseite ist vorzugsweise hydrophob ausgebildet oder weist hydrophobe Abschnitte auf. Ein solcher Abschnitt kann PTFE aufweisen oder daraus bestehen. Die Außenfläche des Rotationselements kann zumindest teilweise oder vollständig von einem hydrophobem Material gebildet sein, insbesondere Polytetrafluorethylen (PTFE). Bei Verwendung von PTFE resultieren gute Eigenschaften der Kontaktseite hinsichtlich der Gleitfähigkeit, mechanischen Haltbarkeit und chemischen Resistenz. Die Kontaktseite ist vorzugsweise planar ausgebildet oder zumindest abschnittsweise planar ausgebildet.

Das Rotationselement kann ein Massenelement aufweisen, das die Gesamtmasse des Rotationselements in definierter Weise erhöht. Das Massenelement kann Metall oder Keramik aufweisen oder daraus bestehen. Da grundsätzlich jedes Bauteil eine Masse aufweist, kann das Massenelement auch noch weitere Funktionen übernehmen, und kann insbesondere der Kopplung zwischen Aktuatoreinrichtung und Rotationselement dienen. Die resultierende Gewichtskraft des Rotationselements kann einen vorbestimmten Druck auf die Kontaktfläche aufbringen oder zusätzlich dazu beitragen. Durch das Massenelement und den vorbestimmten Druck lässt sich gewährleisten, dass das erste Lösungsmittel während der Rotation des Rotationselements in definierter Weise verdrängt wird. Dieser Druck ist ein Parameter bei der erfindungsgemäßen Vorrichtung und dem Verfahren der Herstellung der Molekülschichten, mit dem deren Qualität und Gleichförmigkeit beeinflusst werden kann. Vorzugsweise wird der Druck nur über die Gewichtskraft des Rotationselements erzeugt oder anders erzeugt. Dieser Druck kann z.B. zwischen 0,1 kN/m^2 (Kilo-Newton pro Quadratmeter) und 200 kN/m^2 liegen und liegt vorzugsweise zwischen 1 kN/m^2 und 20 kN/m^2 oder zwischen 2 kN/m^2 und 15 kN/m^2 oder 4 kN/m^2 und 10 kN/m^2, da solche Werte insbesondere zur Erzeugung von Bilipidschichten geeignet sind. Er kann aber auch in bestimmten Fällen höher liegen, vorzugsweise zwischen 20 kN/m^2 und 50 kN/m^2 oder zwischen 50 kN/m^2 und 100 kN/m^2, falls erforderlich.

Das Rotationselement kann einen Wellenverbindungsabschnitt aufweisen, mit dem die rotierbare Welle der Aktuatoreinrichtung mit dem Rotationselement gekoppelt ist, um die Rotation der Welle in eine Rotation des Rotationselements zu wandeln. Der Wellenverbindungsabschnitt kann eine kraftschlüssige und/oder formschlüssige und/oder stoffschlüssige Verbindung der Welle mit dem Rotationselement aufweisen, z.B. als Schraubverbindung, Rastverbindung, Klemmverbindung, Klebe- oder Schweißverbindung. Ferne kann der Wellenverbindungsabschnitt- dazu dienen, außer dem Drehmoment auch noch eine Kraft auf das Rotationselement mit einer zur Rotationsebene senkrechten Komponente zu erzeugen. Die Richtung dieser Kraft kann von der Oberseite des Trägerabschnitts wegzeige oder darauf hin zeigen. Die Kraft kann somit dazu dienen, das Rotationselement "leichter" oder "schwerer" zu machen, und so den Druck zu verringern oder zu erhöhen, mit dem das Rotationselement auf das Trägersubstrat drückt. Der Wellenverbindungsabschnitt kann ferner einen Federabschnitt aufweisen, z.B. einen Verbindungssockel aus thermoelastischem Kunststoff oder einer Metallfeder. Dadurch wird eine Verlagerung der Welle in Richtung des Trägersubstrats zu einem geringeren Druckanstieg zwischen Rotationselement und Trägersubstrat führen, als im Fall einer ungefederten Verbindung. Auf diese Weise kann der Druck besser dosiert werden bzw. Druckschwankungen gedämpft werden. Der Wellenverbindungsabschnitt oder das Federelement lassen vorzugsweise ein Neigen der Welle gegenüber der Normalen eines planaren Abschnitts des Trägersubstrats zu. Auf diese Weise kann das Rotationselement in jeder Winkelstellung der Rotation selbst dann planar auf dem Trägersubstrat aufliegen, wenn die Welle oder das Trägersubstrat geneigt ist.

Die Vorrichtung kann vorzugsweise eine Anzahl N1 Rotationselemente und vorzugsweise eine Anzahl N2 Rotationseinrichtungen aufweisen, wobei N1, N2 > 1, z.B. 1 < N1, N2 < 100, vorzugsweise N1, N2 < 9 oder 17. Auf diese Weise kann ein höherer Durchsatz bei der Herstellung der Molekülschichten erreicht werden. Die Molekülschichtträgervorrichtung kann also mehr als ein Rotationselement aufweisen, und eine Anzahl von Aperturen in dem Trägersubstrat kann vorzugsweise größer oder gleich sein wie die Anzahl der Rotationselemente, so dass das erfindungsgemäße Verfahren wobei das Verfahren auch die folgenden Schritte aufweisen kann: - Bewirken der automatischen Rotation mehrerer Rotationselements oberhalb des Trägersubstrats; - Bewegen des ersten Lösungsmittels oberhalb des Trägersubstrats durch Wechselwirkung der rotierenden Rotationselemente mit dem ersten Lösungsmittel, wodurch sich eine Molekülschicht über mehr als einer Apertur bildet.

Die Aktuatoreinrichtung kann einen Elektromotor aufweisen, um die Rotation des Rotationselements zu bewirken. Der Elektromotor kann direkt oder über eine Getriebeeinrichtung die Rotation bewirken. Eine vom Elektromotor angetriebene Welle kann unmittelbar oder mittelbar mit dem Rotationselement gekoppelt sein. Die Aktuatoreinrichtung kann zur mechanischen Wechselwirkung mit dem Rotationselement ausgebildet sein, um dessen Rotation mechanisch zu bewirken, wobei die Aktuatoreinrichtung vorzugsweise eine rotierbare Welle aufweist, die kinematisch an das Rotationselement gekoppelt ist. Die Welle ist vorzugsweise senkrecht zum Trägersubstrat angeordnet und vorzugsweise mit dessen geometrischen Zentrum oder Masseschwerpunkt verbunden. Es ist aber auch bevorzugt, das Rotationselement über einen ringartige Lagerung rotieren zu lassen, wobei der Ring im wesentlichen parallel zur Rotationsfläche des Rotationselement angeordnet sein kann. Ein Führungselement des Rotationselements wird dann in dieser Lagerung umlaufen und das Rotationselement mitnehmen.

Die Aktuatoreinrichtung kann einen z-Aktuator aufweisen, der das Rotationselement zumindest teilweise in Richtung (definiert als z-Richtung) senkrecht zu einem planaren Abschnitt des Trägersubstrats auslenken kann und so ferner eine Kraft aufbringen kann. Vorzugsweise weist der z-Aktuator einen oder mehrere piezoelektrische Elemente auf oder besteht im wesentlichen daraus. Ferner weist die Aktuatoreinrichtung vorzugsweise einen Kraftaufnehmer auf, mittels dem über die bekannte Größe der Auflagefläche der Kontaktseite des Rotationselements der Druck, jeweils vorzugsweise, ermittelt, kontrolliert, geregelt und/oder dokumentiert werden kann. Der Kraftaufnehmer kann ein piezoresistives Sensorelement sein. Der Kraftaufnehmer kann direkt in die Welle eingefügt sein oder kann mit dem z-Aktuator verbunden sein. Durch einen kontrollierten bzw. geregelten Druck können Varianten der Erfindung besser an individuelle Bedürfnisse bei der Erzeugung von Molekülschichten mit dem erfindungsgemäßen Verfahren und der Vorrichtung ausgerichtet werden.

Es ist aber auch vorzugsweise vorgesehen, dass die Aktuatoreinrichtung zur berührungslosen Wechselwirkung mit dem Rotationselement ausgebildet ist, um dessen Rotation berührungslos zu bewirken, wobei die Aktuatoreinrichtung insbesondere zur Erzeugung eines beweglichen Magnetfeldes ausgebildet ist, durch welches das Rotationselement rotiert wird. Dazu kann in der Aktuatoreinrichtung ein rotierbarer Permanentmagnet vorgesehen sein, z.B. stabförmig oder von anderer Form. Insbesondere im Fall der berührungslosen Wechselwirkung ist das Rotationselement dann ferner dazu ausgebildet, ein Magnetfeld aufzuweisen, so dass das bewegliche Magnetfeld der Aktuatoreinrichtung durch magnetische Wechselwirkung mit dem Magnetfeld des Rotationselements die Bewegung des Rotationselements induziert. Äußerst überraschend hatte sich in Experimentreihen der Erfinder gezeigt, dass eine effektive, erfindungsgemäße Vorrichtung bzw. das Verfahren zur Herstellung der Molekülschicht erzielt werden können, indem ein handelsüblicher magnetischer Rührfisch als Rotationselement verwendet wird, welches nur durch sein geeignetes Eigengewicht auf das Trägersubstrat drückt. Obwohl diese Ausführungsform bevorzugt ist, ist es auch möglich und bevorzugt, dass das Rotationselement weitere vorteilhafte Strukturmerkmale aufweist, die z.B. ein handelsüblicher Rührfisch nicht besitzt.

Vorzugsweise weist das Rotationselement eine Kanaleinrichtung auf, über die eine Flüssigkeit durch das Rotationselement hindurch oder an diesem entlang in Richtung des Trägersubstrats geleitet (z.B. gepresst) oder von diesem weggesaugt werden kann. Die Kanaleinrichtung kann mit einer Ansauganlage verbunden sein, die vorzugsweise Teil der Vorrichtung ist, insbesondere Teil der Aktuatoreinrichtung, und die zum Dosieren dieser Flüssigkeit dienen kann. Die Kanaleinrichtung kann auch als Öffnung im Rotationselement ausgeführt sein, die insbesondere in dessen geometrischem Zentrum oder Masseschwerpunkt liegen kann. Die Kanaleinrichtung kann insbesondere dazu verwendet werden, um das erste Lösungsmittel, welches die amphiphilen Moleküle enthält, zum Trägersubstrat zu führen, insbesondere um dieses direkt zum Zentrum der Rotation zu führen, von wo aus es sich nach außen ausbreitet, zwischen Trägersubstrat und Rotationselement. Dies erfolgt vom Rotationszentrum aus besonders gleichmäßig und effektiv, da die Zentrifugalkraft eine radial nach außen gerichtete Beschleunigung des ersten Lösungsmittels bewirkt, wenn dieses zur Rotation gebracht wird. Die als Öffnung ausgestaltete Kanaleinrichtung kann insbesondere Trichterförmig sein, um das Zugeben einer Flüssigkeit, insbesondere des ersten Lösungsmittels, technisch zu vereinfachen.

Ferner kann das Rotationselement eine Sensorik oder Teile von Messsensorik aufweisen. Insbesondere kann ein Kapazitätsmesser vorgesehen sein, indem an der Kontaktseite des Rotationselements eine Elektrode vorgesehen ist, mit der insbesondere während der Rotation Impedanzmessungen durchgeführt werden. Bei bekanntem Dielektrikum im Spalt kann die Spaltbreite, also der Abstand zwischen Kontaktseite des Rotationselements und Trägersubstrat ermittelt werden; bei bekannter Spaltbreite kann auf das Dielektrikum geschlossen werden, insbesondere die Entstehung einer Molekülschicht beurteilt werden. Ferner kann eine solche Elektrode dazu verwendet werden, um noch während der Rotation den elektrischen Widerstand über die sich bildende Molekülschicht zu einer Gegenelektrode zu überwachen, die auf der anderen Seite der Membran angeordnet sein kann. Die Gegenelektrode kann z.B. unmittelbar auf dem Trägersubstrat angeordnet sein, das von der Molekülschicht bedeckt wird, oder kann in dieses eingearbeitet sein, z.B. in einer elektrolytgefüllten Mikrokavität angeordnet sein, über deren Apertur sich die Molekülschicht ausbildet. Hohe Widerstände z.B. im Bereich hunderter Megaohm oder Gigaohm können als Maß für die Güte der elektrischen Abdichtung und somit die Qualität der gebildeten Molekülschichten, insbesondere der Bilipdschichten, verwendet werden. Eine solche Messsensorik kann auch mindestens eine Komponente für eine optische Messung aufweisen, z.B. Lichtleiter, Spiegel oder Linsen, um z.B. die Rotationsgeschwindigkeit oder die Spaltbreite optisch zu erfassen.

Vorzugsweise weist die Vorrichtung eine elektrische Steuereinrichtung auf, die zur automatischen Steuerung der Aktuatoreinrichtung ausgebildet ist, um insbesondere das Starten, das Stoppen und die Geschwindigkeit der Rotation automatisch zu steuern oder zu regeln. Eine solche Steuereinrichtung kann einen Mikroprozessor aufweisen, sowie Datenspeicher (vom Typ ROM und/oder RAM) und Datenschnittstellen sowie Eingabe und Ausgabegeräte für eine Benutzerschnittstelle. Die Steuereinrichtung kann einen oder mehrere Regelkreise zur Regelung eines beobachtbaren und beeinflussbaren physikalischen Parameters, z.B. des Drucks oder der Rotationsgeschwindigkeit, aufweisen. Die Steuereinrichtung kann programmierbar ausgeführt sein, so dass die Automatisierung der Herstellung der Molekülschichten weiter verbessert werden kann, indem z.B. in Abhängigkeit von Messwerten der vorrichtungseigenen Sensorik oder externer Sensorik die Rotationsgeschwindigkeit, der Druck, oder eine automatische Zugabe von Flüssigkeit, insbesondere des ersten Lösungsmittels automatisch, vorzugsweise programmgesteuert, angepasst werden. Ferner können weitere Messungen an der Membran oder Vorgänge in der Vorrichtung automatisch nach Detektion der erfolgreichen Herstellung der Membran automatisch eingeleitet werden.

Das Verfahren sieht vorzugsweise vor, eine Rotationsgeschwindigkeit des Rotationselements zu verwenden, die vorzugsweise zwischen 0,1 Umdrehungen pro Sekunde (U/s) und 5 U/s liegt. Es hat sich gezeigt, dass vor allem solche relativ langsamen Rotationsgeschwindigkeiten für die Herstellung der Molekülschichten günstig sind. Die Rotationsgeschwindigkeit kann aber auch anders definiert sein, z.B. vorzugsweise kleiner als 3 U/s, 5 U/s, 10 U/s, 20 U/s, 30 U/s, 40 U/s, 50 U/s. Die Steuereinrichtung ist vorzugsweise dazu ausgebildet, eine oder mehrere Rotationsgeschwindigkeiten automatisch einzustellen, d.h. ohne dass eine Benutzerjustierung der Geschwindigkeit erforderlich wäre, oder eine Sequenz von graduell oder kontinuierlich veränderten Geschwindigkeiten programmgesteuert einzustellen, um ein für die individuelle Ausbildung einer Molekülschicht in einem bestimmten Lösungsmittel optimales Geschwindigkeitsmuster in Abhängigkeit von der Zeit einzustellen. Der Benutzer wählt lediglich die gewünschte Geschwindigkeit oder das gewünschte Geschwindigkeitsmuster an, welches dann automatisch von der Vorrichtung eingestellt wird.

Das Verfahren weist vorzugsweise den Schritt auf, dass sich über einer wenigstens einmal vom Rotationselement überstrichenen Oberfläche eine Molekülschicht aus amphiphilen Molekülen ausreichender Qualität ergibt, insbesondere, wenn das Rotationselement eine Umdrehung von mindestens einmal 180° oder 360° ausgeführt hat (also eine vollständige Umdrehung). Dies reichte in einem Ausführungsbeipiel des Verfahrens und der Vorrichtung überraschender Weise bereits aus, um eine Molekülschicht erfolgreich herzustellen. Vorzugsweise sind mindestens 2, 3, 4, 5, 6, 7, 8, 9 oder 10 vollständige Umdrehungen des Rotationselements vorgesehen, vorzugsweise zwischen 1 und 30, zwischen 1 und 20, zwischen 1 und 10, zwischen 1 und 5 oder zwischen 1 und 3 vollständige Umdrehungen. Unter einer Molekülschicht aus amphiphilen Molekülen ausreichender Qualität wird insbesondere eine bimolekulare Schicht (z.B. eine Bilipidschicht) verstanden, bei der die Moleküle in einem Stapel aus zwei Molekülschichten angeordnet sind, der insbesondere durch Selbstorganisation der Moleküle entstanden ist. Eine Molekülschicht aus amphiphilen Molekülen ausreichender Qualität ist ferner vorzugsweise eine im wesentlichen defektfreie Molekülschicht, oder eine Molekülschicht, bei der z.B. mittels einer Messung durch die Spannungsklemmtechnik elektrische Widerstände über die Molekülschicht von mindestens 100 Megaohm, und jeweils vorzugsweise von mindestens 500, 1000, 5000, 10000 Megaohm gemessen werden. Solche Molekülschichten weisen einen hohen "Sealwiderstand" (eine hohe elektrische Dichtigkeit) auf.

Vorzugsweise weist die Vorrichtung mindestens eine Sensoreinrichtung auf, die insbesondere einen Sensor für elektrophysiologische Untersuchungen an der Molekülschicht, insbesondere Bilipidschicht, aufweist. Die Sensoreinrichtung kann eine Elektrode vorsehen, die diesseits der Molekülschicht am Trägersubstrat angeordnet ist, z.B. in einer Mikrokavität, wie eingangs beschrieben, und kann ferner mindestens eine weitere Elektrode auf der anderen Seite der Molekülschicht aufweisen, die im Elektrolyt oberhalb der Molekülschicht angeordnet ist. Diese Sensoreinrichtung ist vorzugsweise zur Durchführung der Spannungsklemmtechnik ausgebildet, mittels der bei konstant gehaltener Spannung kleinste Ströme im Nanoamperebereich und darunter gemessen werden können, insbesondere im Picoamperebereich, z.B. unter Verwendung eines Voltage-Clamp- oder Patch-Clamp-Verstärkers. Die Sensoreinrichtung kann ein Array von Sensoren aufweisen, die im Trägersubstrat oder an dessen Oberfläche angeordnet sein können.

Die Vorrichtung kann eine Transporteinrichtung aufweisen, mittels der das Trägersubstrat und das Rotationselement relativ zueinander bewegbar sind. Vorzugsweise ist das Rotationselement senkrecht zum Trägersubstrat bewegbar, also insbesondere vom Trägersubstrat automatisch entfernbar oder diesem näherbar. Möglich ist, dass eine zweite Transporteinrichtung vorgesehen ist, mittels der das Trägersubstrat oder eine Vielzahl von Trägersubstraten insbesondere parallel zum Trägersubstrat, insbesondere in horizontaler Richtung, bewegt werden, oder das Rotationselement oder die erste Transporteinrichtung in dieser Richtung bewegt werden. Dann könnten z.B. mit hohem Durchsatz auf vielen Trägersubstraten nacheinander Molekülschichten "im Fließbandsystem" erzeugt werden.

Das erste Lösungsmittel zum Lösen der amphiphilen Moleküle und deren Konzentration in dem ersten Lösungsmittel sollten so gewählt sein, dass sich die Moleküle, insbesondere die Lipide, vollständig lösen. Geeignete Parameter sind je nach Einzelfall zu wählen. Zum einen muss das Lösen der Moleküle respektive Lipidmoleküle vollständig gewährt sein, d.h. die kritische Konzentration für eine Mizellenausformung (engl.: Critical Micelle Concentration, CMC) sollte möglichst hoch sein; zum anderen sollte das für das Lösen der Lipide verwendete Lösungsmittel (LM) sehr schlecht bis gar nicht mit wässrigen Lösungen mischbar sein. Geeignete erste Lösungsmittel sind z.B. tendenziell unpolare Stoffe wie z.B. längerkettige Alkane, z.B. mit fünf bis 40 als Kette verbundenen Kohlenstoffen, jeweils besonders vorzugsweise Pentan, Hexan, Heptan, Oktan, Nonan, ferner z.B. auch Dekan, Dodekan, Hexadekan usw. Bevorzugt ist, dass das erste Lösungsmittel eine Mischung aus zwei, drei , vier, oder mehreren der genannten Stoffe aufweist oder daraus besteht. Weitere geeignete erste Lösungsmittel, insbesondere zum Lösen von Lipiden, ergeben sich z.B. aus "Baaken et al" oder US 2009/0167288 A1.

Als zweites Lösungsmittel, nämlich als Elektrolyt, der unterhalb und oberhalb der sich bildenden Molkülschicht platziert wird, kommen insbesondere Salzlösungen in Frage, insbesondere physiologische Salzlösungen, welche die elektrophysiologische Vermessung der Molkülschicht, z.B. Lipid-Membran, und darin enthaltener, ladungstransportierender Poren, z.B. Kanalproteine, erlauben. Geeignete zweite Lösungsmittel, insbesondere zur Durchführung von Messungen mittels Spannungsklemmtechnik, ergeben sich z.B. aus "Baaken et al" oder US 2009/0167288 A1.

Geeignete amphiphile Moleküle zur Herstellung der Molekülschichten sind insbesondere Lipide, insbesondere zur Bildung von membranartigen Doppellipidschichten geeignete Lipide, wie sie z.B. u.a. in"Baaken et al" oder US 2009/0167288 A1 genannt werden.

Von der akademischen Grundlagenforschung bis hin zum pharmakologischen Wirkstoffscreening ist die stabile, einfache, schnelle und zuverlässige Erzeugung einer Lipiddoppelschicht als artifizielles Zellmodell, in verschiedensten Anwendungen ein maßgeblicher Faktor. Für letzere sind insbesondere hohe experimentelle Durchsätze für eine generelle, produktive Entwicklung äußerst wichtig. Anwender aus der Grundlagenforschung profitieren von der Simplifizierung bei experimentellen Durchläufen, von reduziertem apparativem und systemischem Aufwand und von einer hohen Variabilität bei Experimenten mit statistisch unwahrscheinlichen Ereignissen bzgl. der Untersuchung von Membranproteinen oder der Modelmembran selbst. Zusammenfassend ist festzustellen: Die vorliegende Erfindung steigert für Anwender aller elektrophysiologischen und biophysikalischen Fachrichtungen die Effizienz bei Experimenten mit Modelzellmembranen, indem sie generell die Zeitinvestitionen für deren Durchführung deutlich reduziert.

Weitere bevorzugte Ausgestaltungen des erfindungsgemäßen Verfahrens und der erfindungsgemäßen Vorrichtung ergeben sich aus der nachfolgenden Beschreibung der Ausführungsbeispiele in Zusammenhang mit den Figuren. Gleiche Bezugszeichen bezeichnen im wesentlichen gleiche Bauteile.
Fig. 1 zeigt schematisch den Ablauf des erfindungsgemäßen Verfahrens als Ausführungsbeispiel mit Bezug auf die Ausführungsbeispiele der erfindungsgemäßen Vorrichtung in den Figuren 2a bis 4c.
Fig. 2a zeigt ein erstes Ausführungsbeispiel der erfindungsgemäßen Vorrichtung als schematischen Querschnitt.
Fig. 2b zeigt die Vorrichtung der Fig. 2a als isometrische Ansicht.
Fig. 3a zeigt ein zweites Ausführungsbeispiel der erfindungsgemäßen Vorrichtung als schematischen Querschnitt.
Fig. 3b zeigt die Vorrichtung der Fig. 3a als isometrische Ansicht.
Fig. 3c zeigt ein drittes Ausführungsbeispiel der erfindungsgemäßen Vorrichtung als schematischen Querschnitt.
Fig. 3d zeigt die Vorrichtung der Fig. 3c als isometrische Ansicht.
Fig. 4a zeigt ein viertes Ausführungsbeispiel der erfindungsgemäßen Vorrichtung als schematischen Querschnitt.
Fig. 4b zeigt eine Detailansicht aus Fig. 4b.
Fig. 4c zeigt schematisch eine Messanordnung, die mit der erfindungsgemäßen Vorrichtung aus Fig 4a und 4b realisiert wird.
Fig. 5a, 5b, 5c, 5d, 6a, 6b, 6c, 6d, 7a, 7b, 7c und 7d zeigen Messergebnisse an einer Doppellipidschicht, die erfindungsgemäß hergestellt wurde und mittels der in Fig. 4a bis 4c gezeigten Messanordnung gemessen wurde.

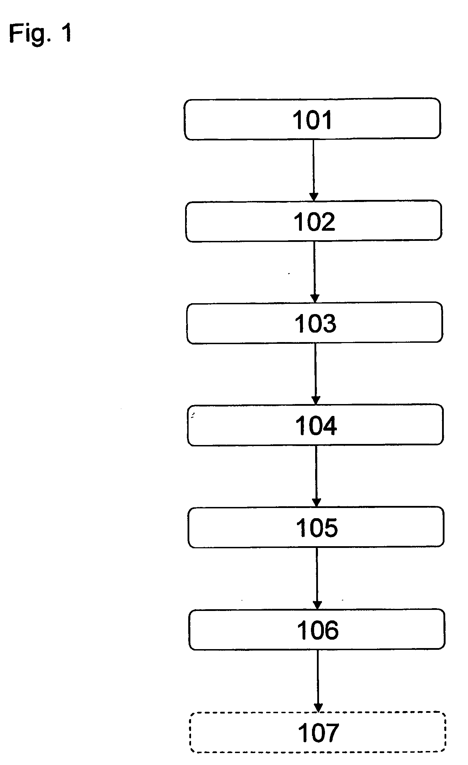

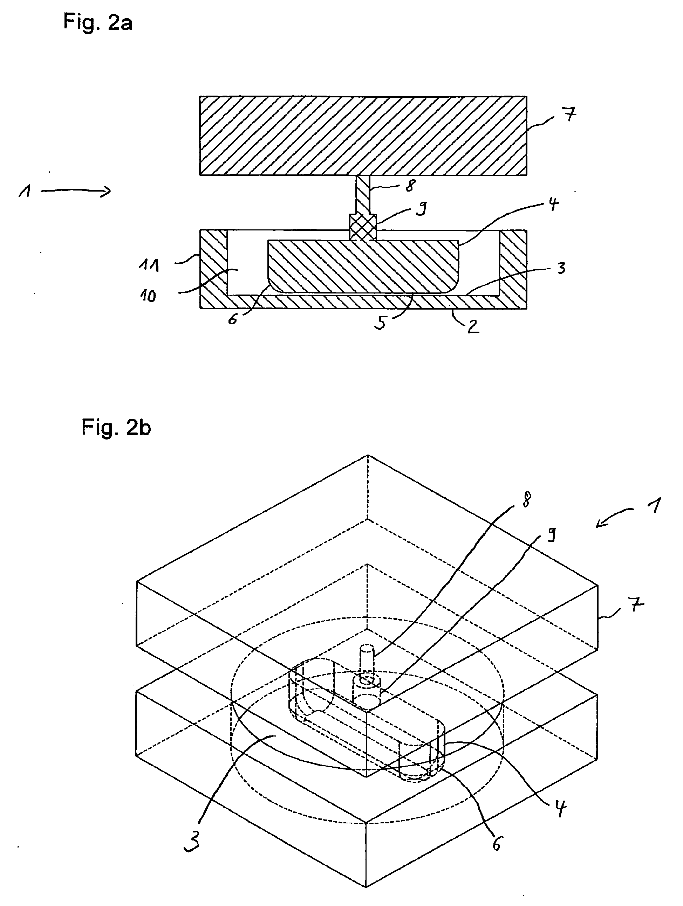

Figur 1 zeigt schematisch die Schritte des Verfahrens, um z.B. mittels einer Vorrichtung nach einer der Figuren 2a, 3a, 3c oder 4a eine Bilipidschicht herzustellen und anschließend zu vermessen.

Die Vorrichtung 1 nach Fig. 2a weist ein Trägersubstrat 2 auf, das eine planare Oberseite 3 aufweist. Diese Oberseite 3 ist mit SU8-Fotolack beschichtet, was für die Ausbildung von Doppelschichten aus amphiphilen Molekülen vorteilhaft ist. Ferner erlaubt dies eine relativ einfache photolitographische Mikrostrukturierung der Oberfläche. In der Oberseite 3 sind Mikrokavitäten der Tiefe 50 µm mit Mikroaperturen (Durchmesser 50 µm) als Array im Abstand von 1 mm angeordnet (in Fig. 2a nicht gezeigt). Die Vorrichtung weist ein Rotationselement 4 auf, das an seiner Unterseite eine planare Kontaktseite 5 aufweist. Das Rotationselement ist ein stabförmiges Teil, das in Richtung der Unterseite abgerundete Kanten 6 aufweist. Die Außenfläche des Rotationselements ist im Bereich seiner Seitenflächen 6 und der Kontaktseite 5 mit PTFE beschichtet. Dadurch ist es in diesem Bereich hydrophob, was für die Herstellung von Doppelschichten aus amphiphilen Molekülen wichtig ist.

Die Vorrichtung 1 der Fig. 2a weist ferner eine Aktuatoreinrichtung 7 auf, die einen Elektromotor enthält, der die starre Welle 8 rotiert. Die Welle 8 ist über einen Wellenverbindungsabschnitt 9 mit dem Rotationselement gekoppelt. Dieser ist als Federelement 9 ausgebildet und besteht aus thermoelastischem Kunststoff. Auf diese Weise kann der Druck des Rotationselements 4 auf die Oberseite 3 des Trägersubstrats besser eingestellt werden. Der vorliegende Druck ergibt sich aus der Auslenkung des am Federelement 9 hängenden Rotationselements und liegt ungefähr bei 5 kN/m^2.

Die Vorrichtung 1 weist über dem Trägersubstrat 2 eine flüssigkeitshaltende Kammer 10 auf, die vom Trägersubstrat und Wandabschnitten 11 gebildet wird.

Fig. 2b verdeutlicht die räumliche Ausgestaltung der Vorrichtung nach Fig. 2a in einer isometrischen Ansicht. Gestrichelte Linien sind eigentlich verborgen und deuten die Bereiche an, die in dieser isometrischen Ansicht eigentlich nicht sichtbar wären.

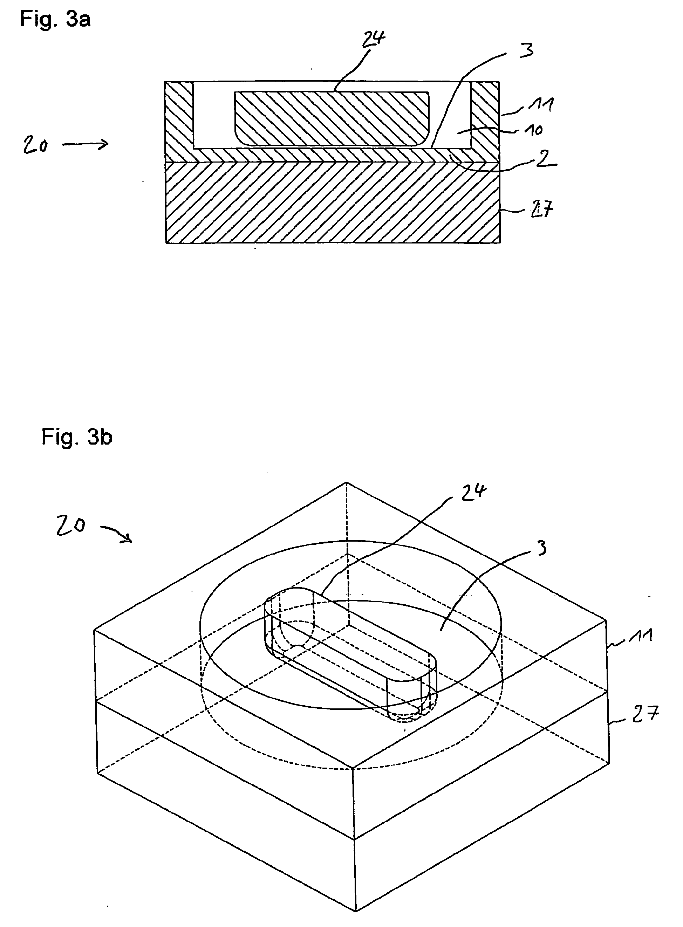

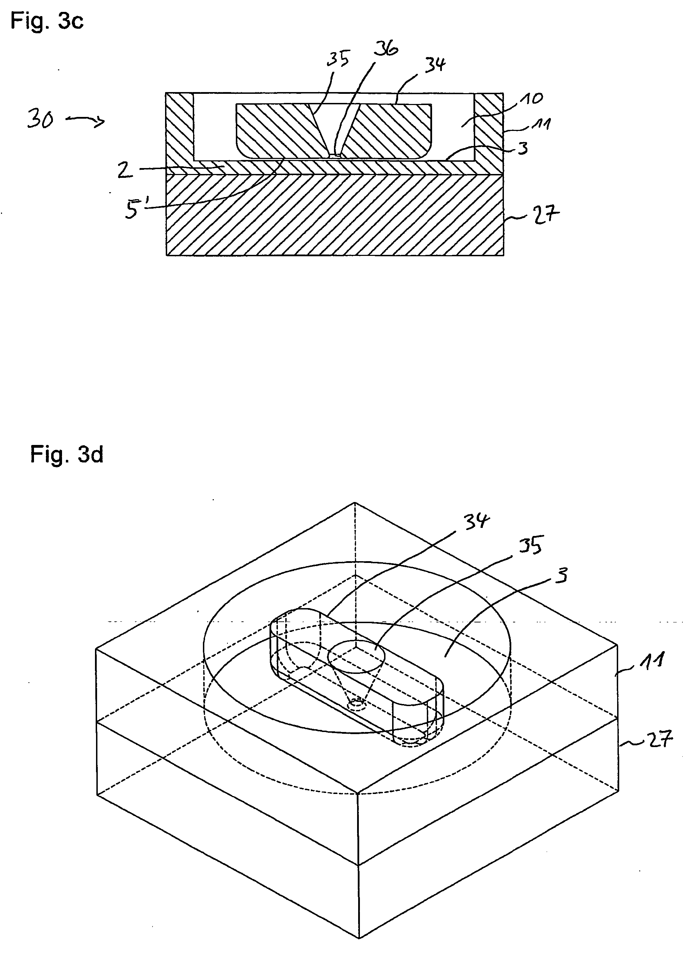

Wie in Fig. 1 gezeigt ist, sieht das Verfahren zur Herstellung der Molekülschicht vor, zunächst eine Lösung eines amphiphilen Moleküls, hier eines Lipids, z.B. Diphytanolphosphoditylcholin, in einem geeigneten ersten Lösungsmittel, z.B. Dekan, bereitzustellen, in einer Konzentration, bei der eine vollständige Lösung möglich ist, hier z.B. 1 mg/ml (Schritt 101). Ein zweites Lösungsmittel, der wässrige, elektrisch leitfähige Elektrolyt, in dem Salze gelöst sind, wird in den Bereich der Kammer 10 (Fig. 2a) oberhalb des Trägersubstrats 2 eingebracht (Schritt 102). Dann werden wenige Mikroliter des zweiten Lösungsmittels, welches das Lipid enthält, mittels eines Kapillarkörpers über der Oberseite 3 und den Mikrokavitäten des Trägersubstrats abgesetzt (Schritt 103). Aufgrund der schlechten Löslichkeit des ersten und zweiten Lösungsmittels kommt es nicht zur Durchmischung. Der Kapillarkörper kann dabei manuell oder vorzugsweise automatisch bewegt werden. Der Kapillarkörper kann eine Glaskapillare, eine Pipettenspitze oder ein sonstiges Kanalelement sein, mit dem durch Anlegen von Druck das in der Kapillare gespeicherte erste Lösungsmittel abgegeben werden kann. Der Kapillarkörper kann als Teil einer Kanaleinrichtung im Rotationselement integriert sein (siehe nachfolgend Fig. 3c). Dann wird die Aktuatoreinrichtung 7 mit Rotationselement 4 in eine vorbestimmte Position gebracht, in der das Rotationselement den vorbestimmten Druck auf das Trägersubstrat 2 ausübt (Schritt 104) und die zuvor ermittelt wurde. Das erste Lösungsmittel befindet sich in einem Spalt zwischen der Kontaktseite 5 des Rotationselements 4 und der Oberseite 3 des Trägersubstrats 2 (siehe Fig. 2a). Nun wird das Rotationselement automatisch mit vorbestimmter Geschwindigkeit, z.B. 2,5 bis 3, 5 U/s rotiert (Schritt 105). Die Bewegung des ersten Lösungsmittels auf der Oberseite 3 oberhalb der Mikroaperturen und Mikrokavitäten begünstigt nun überraschend erfolgreich die Ausbildung von Doppellipidschichten über den Mikroaperturen, die dort BLM bilden (Schritt 106).

Grund für den Erfolg des erfindungsgemäßen Verfahrens und die Vorrichtung sind die definierte und gleichförmige Bewegung des Lipids durch den hydrophoben mechanischen Rotationskörper, der das erste Lösungsmittel dort automatisch besonders gleichförmig aufbringt bzw. ideal spreitet. Die Tatsache, dass die Lipidlösung durch die Drehbewegung zwischen zwei ähnlich hydrophoben Oberflächen (hier SU8 und PTFE) gespreitet wird, begünstigt weiter den Erfolg der Ausbildung der Doppellipidschicht. Gegenüber der händischen Painting-Methode entfällt die Wartezeit von typischerweise einigen Sekunden bis zur Ausbildung eines Bilayers. Bei der vorliegenden Erfindung bilden sich die Doppelschichten vielmehr innerhalb der ersten Sekunde der Rotation.

Fig. 3a zeigt eine Vorrichtung 20, bei der der Rotationskörper 24 den Rotationskörper 4 der Fig. 2a ersetzt. Rotationselement 24 weist dieselben Eigenschaften (Form, PTFE-Beschichtung) wie Rotationselement 4 auf, weist aber anstelle der mechanischen Verbindung mit der Aktuatoreinrichtung, wie als Welle 8 und Wellenverbindungsabschnitt 9 in Fig. 2a gezeigt, Mittel zur berührungsfreien Kopplung mit der entsprechenden Aktuatoreinrichtung 27 auf. Rotationselement 24 weist dazu einen Kern aus einem stabförmigen Permanentmagneten auf, dessen Magnetfeld mit dem von der Aktuatoreinrichtung 27 erzeugten rotierenden Magnetfeld wechselwirkt, um das Rotationselement 24 zu rotieren. Die Aktuatoreinrichtung 27 weist dazu einen Gleichstrom-Elektromotor auf, der einen innerhalb der Aktuatoreinrichtung angeordneten Stabmagneten mit der gewünschten Geschwindigkeit rotiert. Diese Geschwindigkeit entspricht der für die Rotation des Rotationselements 24 gewünschten Rotationsgeschwindigkeit. Durch die berührungsfreie Einkopplung der Rotation ist eine noch gleichmäßigere Rotationsbewegung möglich, was die Ausbildung der Bilipdschichten (BLM) weiter begünstigt.

Fig. 3c zeigt eine Vorrichtung 30, die der Vorrichtung 20 in Fig. 3a bis auf das anders ausgebildete Rotationselement 34 gleicht. Das Rotationselement 34 weist hier eine als trichterförmige Öffnung 35 ausgebildete Kanaleinrichtung auf, mittels der das erste Lösungsmittel durch das Rotationselement 34 zur Oberseite 3 des Trägersubstrats 2 geleitet werden kann. Die Kanaleinrichtung mündet über den sich an der Kontaktseite 5' des Rotationselements 34 wieder verbreiternden Mündungsbereich 36 direkt im geometrischen Rotationszentrum des Rotationselements 34. Wird das erste Lösungsmittel durch diesen Kanal zur Oberseite 3 des Trägersubstrats gepresst, kann es sich von dort durch die Rotation begünstigt radial nach außen ausbreiten.

Fig. 4a bis Fig. 4c zeigen, wie nach dem Herstellen der Bilipidschicht auf der Oberseite 3 des Trägersubstrats 2 Messungen an der Bilipidschicht durchgeführt werden können, um insbesondere die Qualität der Bilipidschicht zu beurteilen, in einem optionalen Schrit 107. Dies gilt für alle in Fig. 2a bis 3d gezeigten Vorrichtungen. Oberhalb der nach oben in offene Mikroaperturen mündenden Mikrokavitäten 41 haben sich durch das Verfahren Lipide zu einer Doppellippidschicht ausgebildet. Jede Mikrokavität 41 ist mit einem Elektrolyt 47 gefüllt und in der Kammer 10 befindet sich der Elektrolyt 48. Jede Mikrokavität wird am Boden nach unten durch eine innere Redoxelektrode 42 (z.B. Ag/AgCl) abgeschlossen. Diese ist über eine z.B. lithographisch erzeugte Zuleitung 43 elektrisch mit der äußeren Kontaktierung 44 verbunden, über die Sensorik verbunden werden kann. Im Elektrolyt 48 der Kammer 10 befindet sich die Redoxelektrode 45, die als Referenzelektrode für jede der in den einzelnen Mikrokavitäten angeordneten Elektroden dient. Durch Spannungsklemmtechnik, bei der eine elektrische Spannung über die Membran z.B. mithilfe eines Patch-Clamp-Verstärkers konstant gehalten wird, können geringste Stromänderungen eines Stroms von Ladungen "q" durch die Membran detektiert werden. Diese Ladungen können auf dem lonentransport durch undichte Stellen der Zellmembran beruhen oder auf Poren, z.B. Kanalproteine 51 in der Zellmembran zurückgehen.

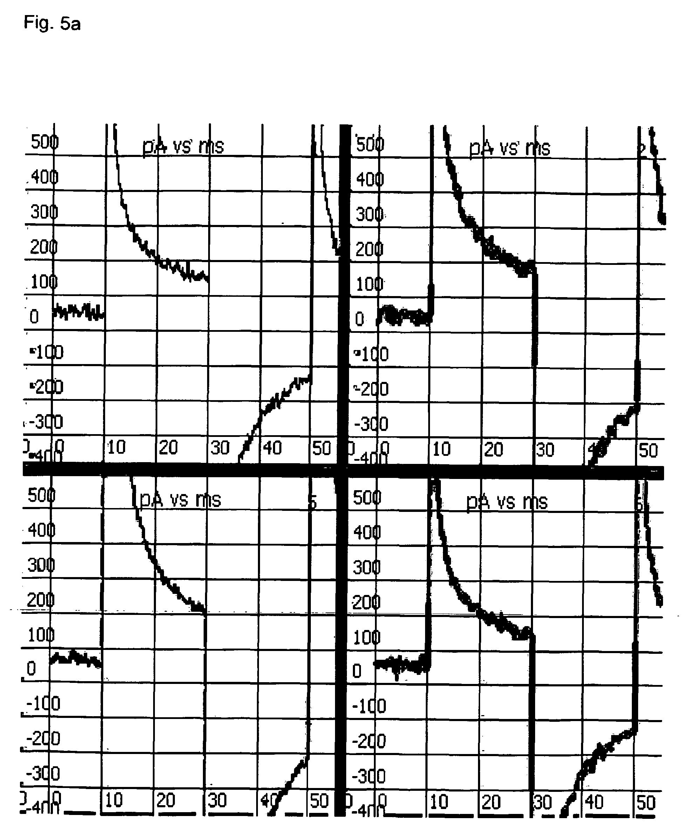

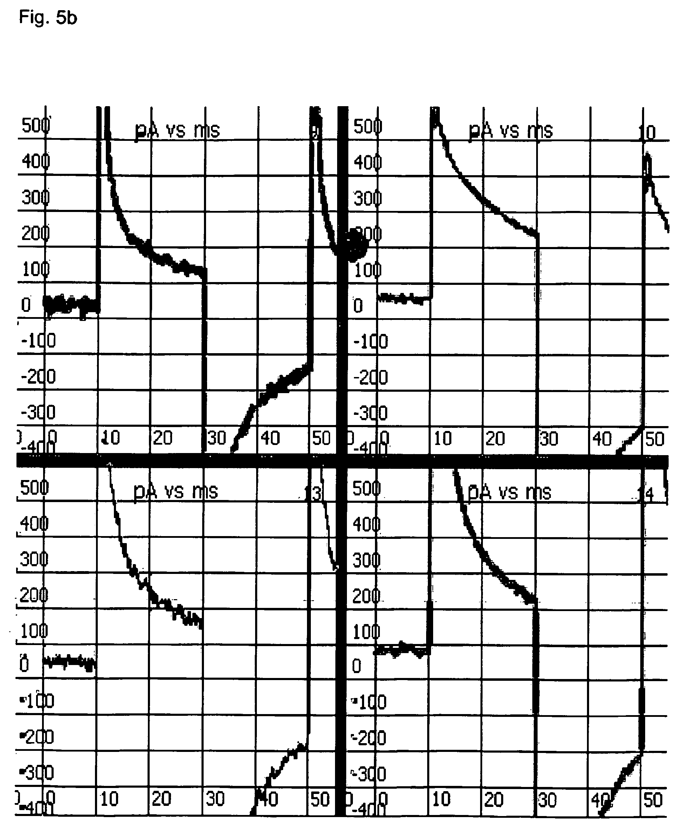

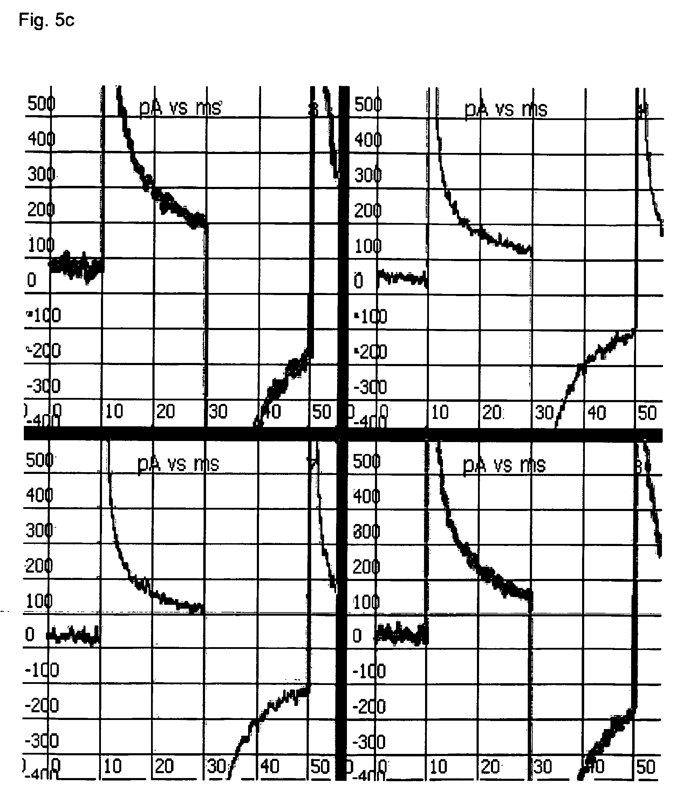

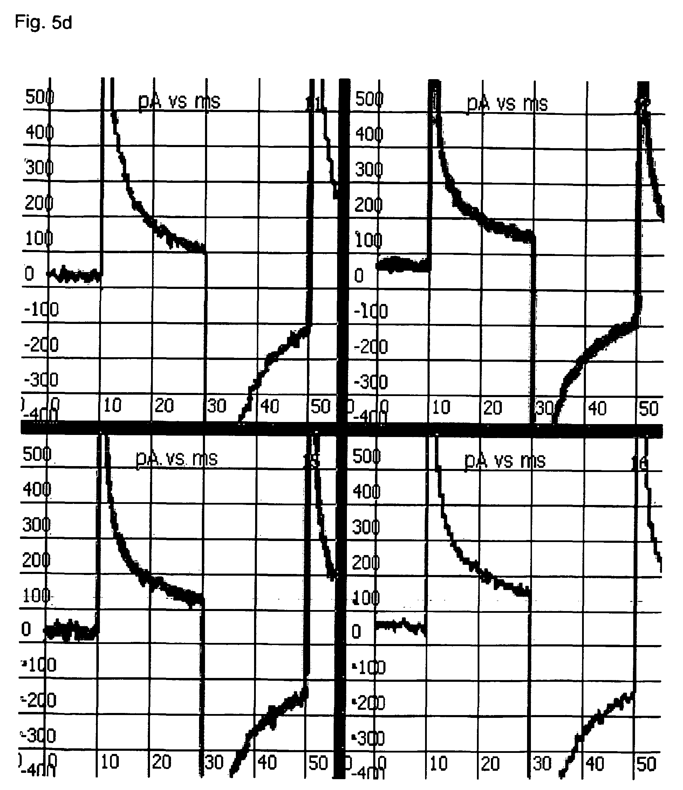

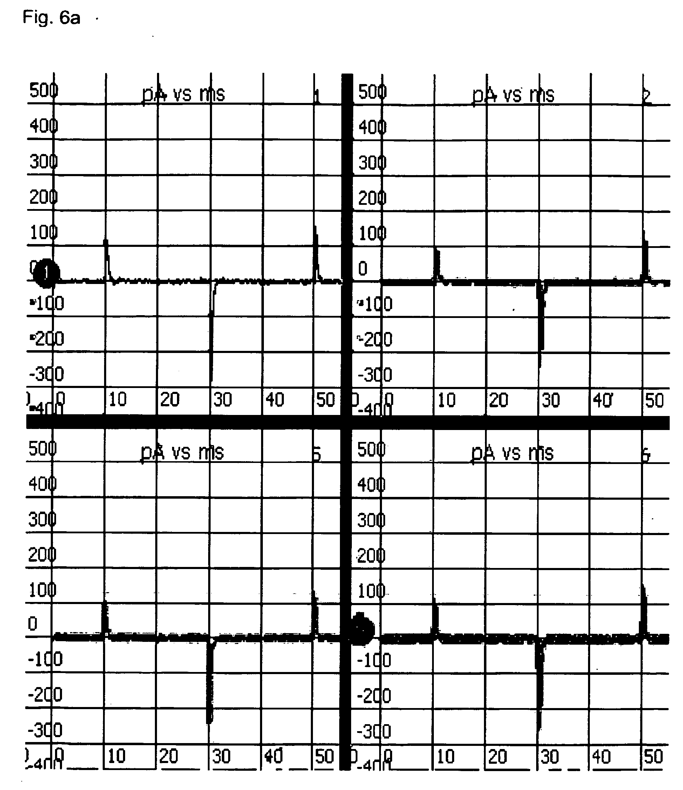

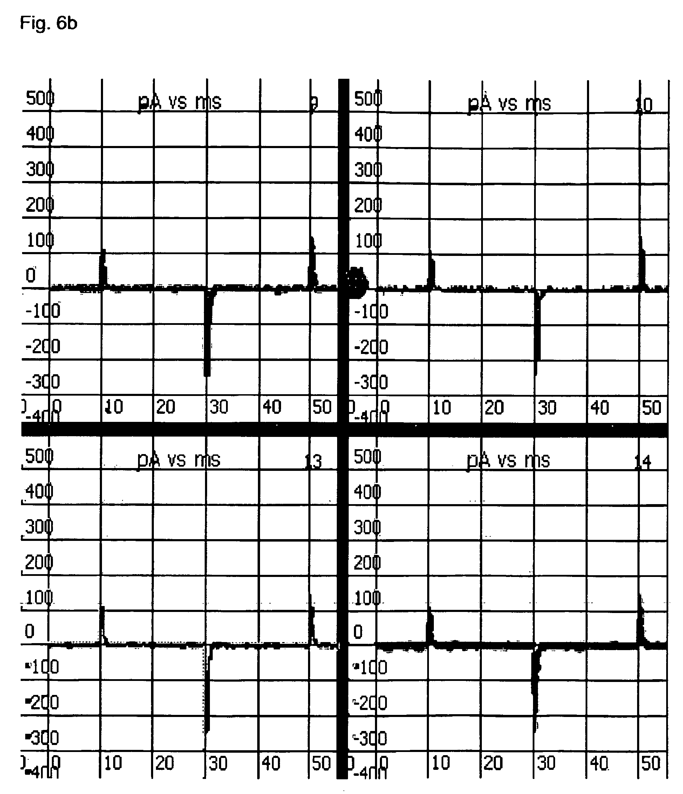

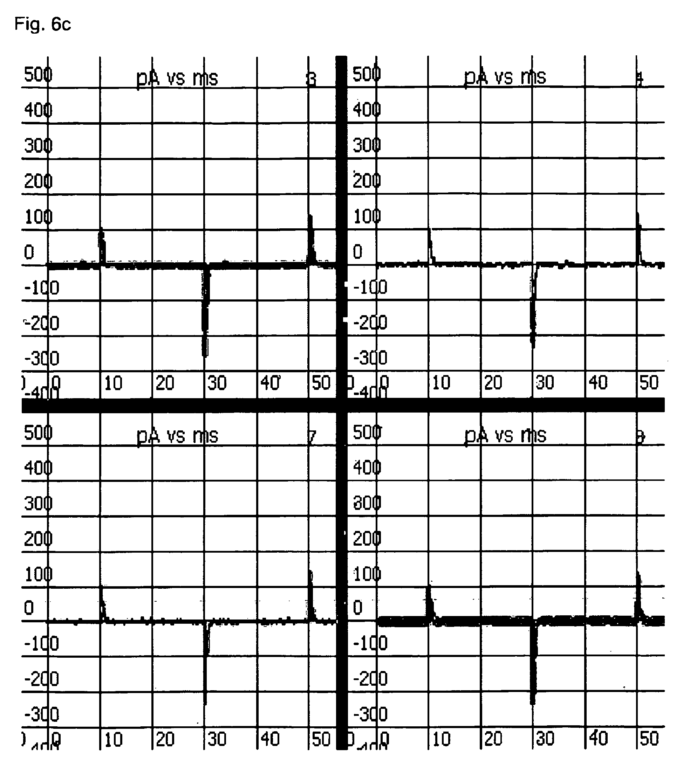

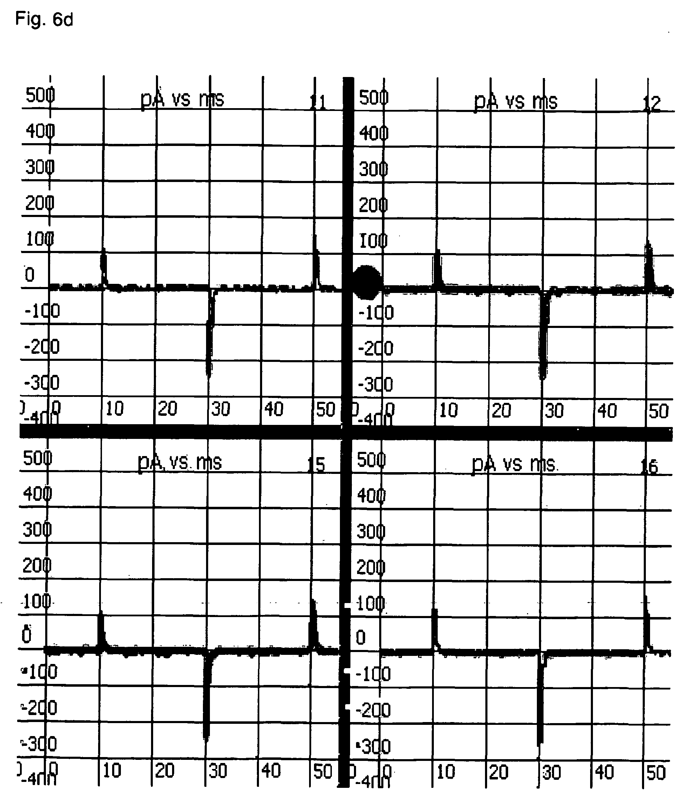

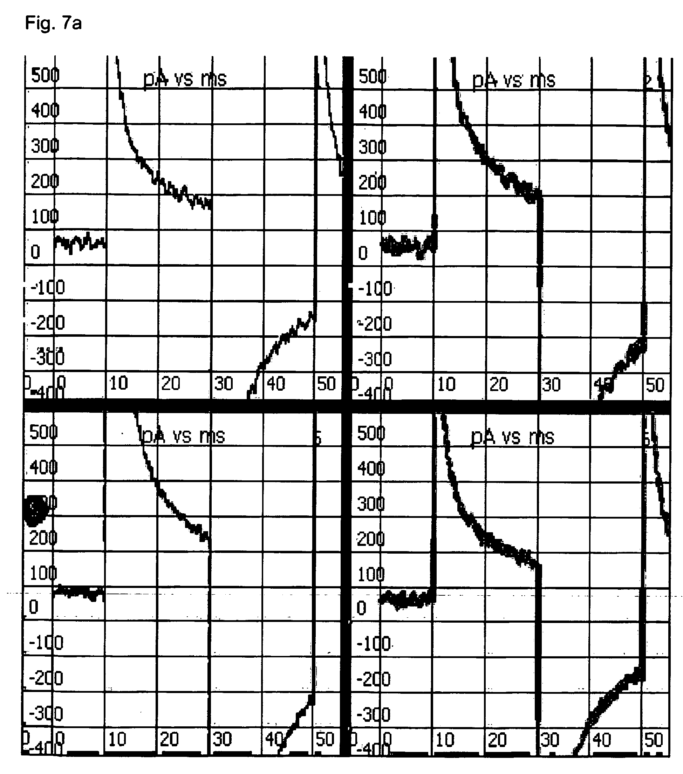

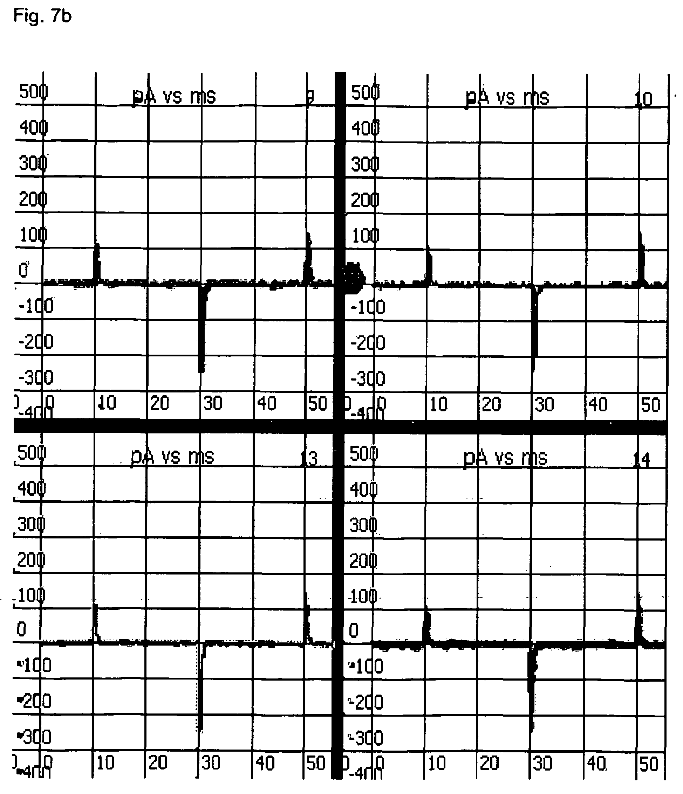

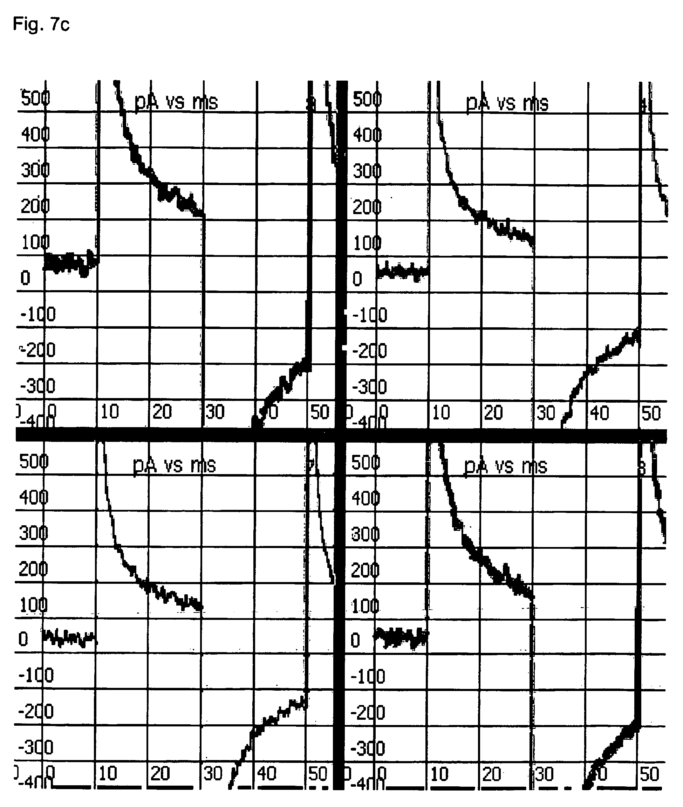

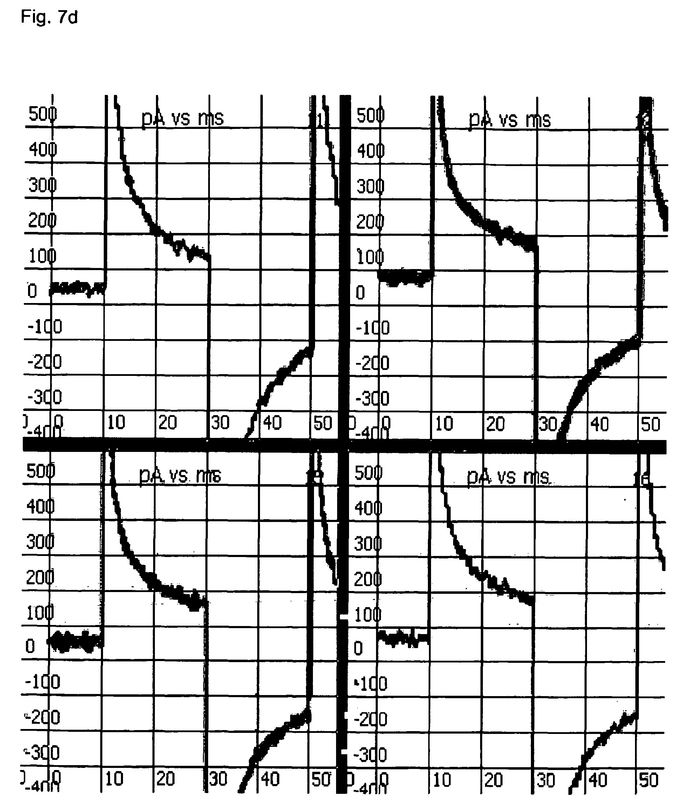

Fig. 5a bis 7d zeigen Messergebnisse von Messungen, die mit einem Aufbau wie in Fig. 4a- 4c gezeigt, ermittelt wurden. Die simultane Ausbildung von Bilipidschichten auf 16 Mikroelektrodenkavitäten (MECA) erfolgt durch einmaliges Rotieren des Rotationselementes über diesen Mikroelektrodenkavitäten, bzw. durch eine einmaliges Überstreichen der Mikroaperturen der Mikroelektrodenkavitäten durch das Rotationselement. Gezeigt sind drei Screenshots des Steuerungsprogramms für den 16-Kanalverstärker Tecella-Jet mit 16 Fenstern, in denen jeweils die Stromverläufe an den einzelnen Mikroelektrodenkavitäten über die Zeit aufgezeichnet werden. Jedes der 16 Einzeldiagramme der Fig. 5a, 5b, 5c, 5d, ebenso 6a, 6b, 6c, 6d, und ebenso 7a, 7b, 7c und 7d zeigt ein Strom-ZeitDiagramm (pA vs. ms) mit einer Ordinatenskalierung von -300 bis 500 pA und einer Abszissenskalierung von 0 bis 50 ms. Fig. 5a, 5b, 5c, 5d zeigt jeweils die Stromantworten vor Betätigung des Rotationselementes; deutlich erkennbar sind die großen Stromänderungen im Größenordnungsbereich von Nanoampere als Antwort auf ein biphasisches (positiv/negatives) Spannungskommando. Fig. 6a, 6b, 6c, 6d zeigt jeweils, dass unmittelbar nach Beendigung der Bewegung alle Mikrokavitäten elektrisch dicht verschlossen sind; sichtbar bleiben allein kapazitive Artefakte im Bereich von wenigen hundert Picoampere. Der Membranwiderstand ist sehr hoch (Gigaohmbereich), es findet "kein" Ladungstransport durch die Membran statt. Fig. 7a, 7b, 7c und 7d zeigt jeweils, dass nach Elektroporation mit einem Spannungspuls von 990 mV Amplitude und 100 ms Dauer die Kavitäten wiederum geöffnet sind. Ein solches Verhalten ist beweisend für die Existenz einer bimolekularen Lipidschicht, da nur hier ausreichend hohe Feldstärken für eine Elektroporation erreicht werden. Die durch die Erfindung herstellbaren Membranen mit erzielten hohen Membranwiderständen sind ideal für Anwendungen, in denen geringste Transmembranströme detektiert werden sollen, um z.B. die Aktivität einzelner ladungstransportierender Transmembranproteine zu untersuchen.

## Patentansprüche

1. Verfahren zur automatisierten Herstellung einer Molekülschicht aus amphiphilen Molekülen, insbesondere Lipiden für eine Bilipidmembran, in einer Vorrichtung (1; 20; 30), die ein Trägersubstrat (2) zum Tragen der Molekülschicht aufweist, ein Rotationselement (4; 24; 34), das über dem Trägersubstrat rotierbar ist, und eine Aktuatoreinrichtung (7; 27), mittels der das Rotationselement automatisch rotierbar ist, wobei das Rotationselement eine Außenfläche aufweist, die zumindest abschnittsweise hydrophob ausgebildet ist, mit den folgenden Verfahrensschritten:
- Einbringen eines ersten Lösungsmittels, welches amphiphile Moleküle enthält, in einen Bereich oberhalb des Trägersubstrats;
- Bewirken der automatischen Rotation des Rotationselements oberhalb des Trägersubstrats;
- Bewegen des ersten Lösungsmittels zwischen dem Trägersubstrat und dem Rotationselement durch Wechselwirkung des rotierenden Rotationselements mit dem ersten Lösungsmittel, wodurch sich die Molekülschicht bildet.

2. Verfahren gemäß Anspruch 1, wobei das ein Trägersubstrat planar ist und eine Oberseite mit mindestens einer Apertur aufweist, oder vorzugsweise ein Array von Aperturen aufweist, und wobei das Verfahren den Schritt aufweist, dass sich die Molekülschicht so bildet, dass sie die mindestens eine Apertur überdeckt.

3. Verfahren gemäß Anspruch 1 oder 2, wobei das Trägersubstrat mindestens eine Mikrokavität aufweist, oder vorzugsweise ein Array von Mikrokavitäten aufweist, wobei eine oder jede Mikrokavität nach oben offen ist und in einer Apertur in der Oberseite des Trägersubstrats mündet, insbesondere einer Apertur gemäß Anspruch 2, und wobei das Verfahren den Schritt aufweist, dass sich die Molekülschicht so bildet, dass sie die mindestens eine Apertur oder mehrere Aperturen überdeckt.

4. Verfahren gemäß wenigstens einem der vorherigen Ansprüche 2 oder 3, wobei die Vorrichtung ein oder mehr als ein Rotationselement aufweist, wobei die Anzahl der Aperturen in dem Trägersubstrat vorzugsweise größer oder gleich ist als die Anzahl der Rotationselemente, und wobei das Verfahren die folgenden Schritte aufweist:
- Bewirken der automatischen Rotation mehrerer Rotationselements oberhalb des Trägersubstrats;
- Bewegen des ersten Lösungsmittels oberhalb des Trägersubstrats durch Wechselwirkung der rotierenden Rotationselemente mit dem ersten Lösungsmittel, wodurch sich eine Molekülschicht über mehr als einer Apertur bildet.

5. Verfahren gemäß wenigstens einem der vorherigen Ansprüche, wobei die Aktuatoreinrichtung zur mechanischen Wechselwirkung mit dem Rotationselement ausgebildet ist, um dessen Rotation mechanisch zu bewirken, wobei die Aktuatoreinrichtung insbesondere eine rotierbare Welle aufweist, die kinematisch an das Rotationselement gekoppelt ist.

6. Verfahren gemäß wenigstens einem der vorherigen Ansprüche 1 bis 4, wobei die Aktuatoreinrichtung zur berührungslosen Wechselwirkung mit dem Rotationselement ausgebildet ist, um dessen Rotation berührungslos zu bewirken, wobei die Aktuatoreinrichtung insbesondere zur Erzeugung eines beweglichen Magnetfeldes ausgebildet ist, durch welches das Rotationselement rotiert wird.

7. Verfahren gemäß wenigstens einem der vorherigen Ansprüche, wobei die Außenfläche des Rotationselements zumindest teilweise oder vollständig von einem hydrophobem Material gebildet wird, insbesondere Polytetrafluorethylen (PTFE).

8. Verfahren gemäß wenigstens einem der vorherigen Ansprüche, wobei die Molekülschichtträgervorrichtung eine elektrische Steuereinrichtung aufweist, die zur automatischen Steuerung der Aktuatoreinrichtung ausgebildet ist, um insbesondere das Starten, das Stoppen und die Geschwindigkeit der Rotation automatisch zu steuern.

9. Verfahren gemäß wenigstens einem der vorherigen Ansprüche, wobei die Vorrichtung mindestens eine Sensoreinrichtung aufweist, die insbesondere einen Sensor für elektrophysiologische Untersuchungen an der Molekülschicht, insbesondere Bilipidschicht, aufweist.

10. Vorrichtung (1; 20; 30) zur automatisierten Bereitstellung einer Molekülschicht (50) aus amphiphilen Molekülen auf einem Trägersubstrat (2) mittels dem Verfahren nach Anspruch 1, aufweisend:
ein Trägersubstrat (2) zum Tragen der Molekülschicht,
ein Rotationselement (4; 24; 34), das über dem Trägersubstrat rotierbar angeordnet ist, und
eine Aktuatoreinrichtung (7;27), die das Rotationselement automatisch rotiert,
wobei das Rotationselement eine Außenfläche aufweist, die zumindest abschnittsweise hydrophob ausgebildet ist,
so dass ein Lösungsmittel, das die amphiphilen Moleküle enthält, bei einer Rotation mittels des Rotationselements auf dem Trägersubstrat bewegt wird, wodurch sich die Molekülschicht ausbreitet.

## Claims

1. A method for the automated fabrication of a molecular layer made from amphiphilic molecules, in particular lipids for a lipid bilayer membrane, in an apparatus (1; 20; 30) that comprises a support substrate (2) for supporting the molecular layer, a rotation element (4; 24; 34) that is rotatable on the support substrate, and an actuator device (7; 27) by means of which the rotation element is automatically rotatable, wherein the rotation element comprises an outer surface that is, at least in sections, hydrophobic, comprising the following method steps:
- placing a first solvent containing amphiphilic molecules in a region above the support substrate;
- effectuating the automatic rotation of the rotation element above the support substrate;
- moving of the first solvent between the support substrate and the rotation element by the interaction of the rotating rotation element with the first solvent, thereby forming the molecular layer.

2. A method according to claim 1, wherein the support substrate is planar and comprises an upper side with at least one aperture, or, preferably, with an array of apertures, and wherein the method comprises the step that the molecular layer forms such that it covers the at least one aperture.

3. A method according to claim 1 or 2, wherein the support substrate comprises at least one microcavity, or preferably, an array of microcavities, wherein one or each microcavity opens upwards and ends in an aperture in the upper side of the support substrate, in particular in an aperture according to claim 2, and wherein the method comprises the step that the molecular layer forms such that it covers the at least one aperture or multiple apertures.

4. A method according to at least one of the preceding claims 2 or 3, wherein the apparatus comprises one or more than one rotation element, wherein the number of apertures in the support substrate is preferably greater than or equal to the number of the rotation elements, and wherein the method comprises the following steps:
- effectuating the automatic rotation of multiple rotation elements above the support substrate,
- moving of the first solvent above the support substrate by the interaction of the rotating rotation elements with the first solvent, thereby forming a molecular layer on more than one aperture.

5. A method according to at least one of the preceding claims, wherein the actuator device is configured to mechanically interact with the rotation element in order to mechanically effectuate its rotation, wherein the actuator device particularly comprises a rotatable shaft that is kinematically coupled to the rotation element.

6. A method according to at least one of the preceding claims 1 to 4, wherein the actuator device is configured for the contactless interaction with the rotation element in order to effectuate its rotation without contact, wherein the actuator device is particularly configured for the generation of a moving magnetic field, by which said rotation element is rotated.

7. A method according to at least one of the preceding claims, wherein the outer surface of the rotation element is at least partially or completely formed by a hydrophobic material, in particular polytetrafluoroethylene (PTFE).

8. A method according to at least one of the preceding claims, wherein the apparatus for supporting the molecular layer comprises an electrical control device that is configured for the automatic control of the actuator device, in particular, in order to automatically control the starting, the stopping, and the speed of the rotation.

9. A method according to at least one of the preceding claims, wherein the apparatus comprises at least one sensor device that comprises, in particular, a sensor for electrophysiological measurements at the molecular layer, in particular the lipid bilayer.

10. An apparatus (1; 20; 30) for the automated preparation of a molecular layer (50) made from amphiphilic molecules on a support substrate (2) by means of the method according to claim 1, comprising:
a support substrate (2) to support the molecular layer,
a rotation element (4; 24; 34) that is rotatably arranged on top of the support substrate, and
an actuator device (7; 27) that rotates the rotation element autamafically,
wherein the rotation element comprises an outer surface that is, at least in sections, hydrophobic,
such that a solvent comprising the amphiphilic molecules is moved on the support substrate at a rotation by meant of the rotation element, thereby spreading the molecular layer.

## Revendications

1. Procédé pour la réalisation automatisée d'une couche moléculaire de molécules amphiphiles, notamment des lipides pour une membrane bilipidique ou bicouche lipidique, dans un dispositif (1; 20; 30) qui comporte un substrat de support (2) pour supporter la couche moléculaire, un élément rotatif (4; 24; 34) susceptible de tourner au-dessus du substrat de support, et un système d'actionneur (7; 27) au moyen duquel il est possible de faire tourner automatiquement l'élément rotatif, l'élément rotatif présentant une surface extérieure, qui est réalisée de marnière à être hydrophobe au moins en partie, le procédé comprenant les étapes de procédé suivantes :
- introduction d'un premier solvant qui renferme des molécules amphiphile, dans une zone au-dessus du substrat de support ;
- production de à rotation automatique de l'élément rotatif au-dessus du substrat de support ;
- mise en mouvement du premier solvant entre le substrat de support et l'élément rotatif par interaction de l'élément rotatif en rotation avec le premier solvant, ce qui conduit à la formation de la couche moléculaire.

2. Procédé selon la revendication 1, d'après lequel ledit substrat de support est plan et présente une face supérieure avec au moins un orifice, ou présente de préférence un réseau d'orifices, et le procédé comprend l'étape selon laquelle la couche moléculaire se forme de manière à recouvrir ledit au moins un orifice.

3. Procédé selon la revendication 1 ou la revendication 2, d'après lequel le substrat de support présente au moins une microcavité, ou présente de préférence un réseau de microcavités, une ou chaque microcavité étant ouverte vers le haut et débouchant dans un orifice dans la face supérieure du substrat de support, notamment dans un orifice selon la revendication 2, et le procédé comprend l'étape selon laquelle la couche moléculaire se forme de manière recouvrir ledit au moins un orifice ou plusieurs orifices.

4. Procédé selon l'une au moins des revendications précédentes 2 ou 3, d'après lequel le dispositif comprend une ou plus d'un élément rotatif, le nombre d'orifices dans le substrat de support étant de préférence supérieur ou égal au nombre des éléments rotatifs, et le procédé présentant les étapes suivantes :
- production de la rotation automatique de plusieurs éléments rotatifs au-dessus du substrat de support ;
- mise en mouvement du premier solvant au-dessus du substrat de support par interaction des éléments rotatifs en rotation, avec le premier solvant, ce qui conduit à la formation d'une couche moléculaire au-dessus de plus d'un orifice.

5. Procédé selon l'une au moins des revendications précédentes, d'après lequel le système d'actionneur est configuré pour l'interaction mécanique avec l'éléments rotatif, pour produire la rotation mécanique de celui-ci, le système d'actionneur comprenant notamment un arbre rotatif, qui est couplé cinématiquement à l'élément rotatif.

6. Procédé selon l'une au moins des revendications précédentes 1 à 4, d'après lequel le système d'actionneur est configuré pour l'interaction sans contact avec l'élément rotatif, pour produire la rotation de celui-ci sans contact, le système d'actionneur étant conçu notamment pour produire un champ magnétique mobile faisant tourner l'élément rotatif.

7. Procédé selon l'une au moins des revendications précédentes, d'après lequel la surface extérieure de l'élément rotatif est formée au moins partiellement ou en totalité par une matériau hydrophobe, notamment du polytétrafluoroéthylène (PTFE).

8. Procédé selon l'une au moins des revendications précédentes, d'après lequel le dispositif de support de couche moléculaire présente un dispositif de commande électrique, qui est conçu pour la commande automatique du système d'actionneur, en vue de commander automatiquement en particulier le démarrage, l'arrêt et la vitesse de la rotation.

9. Procédé selon l'une au moins des revendications précédentes, d'après lequel le dispositif comprend au moins un système de capteur, qui comporte notamment un capteur destiné à effectuer des analyses ou examens électro-physiologiques sur la couche moléculaire, notamment la couche bilipidique.

10. DispositiF (1; 20; 30) pour la préparation automatisée d'une couche moléculaire (50) de molécules amphiphiles sur un substrat de support (2) au moyen du procédé selon la revendication 1, comprenait :
un substrat de support (2) pour supporter la couche moléculaire,
un élément rotatif (4; 24; 34), qui est agencé de manière à pouvoir tourner au-dessus du substrat de support, et
un système d'actionneur (7; 27), qui fait tourner l'élément rotatif de manière automatique,
l'élément rotatif présentant une surface extérieure, qui est réalisée de manière à être hydrophobe au moins en partie,
de sortie qu'un solvant, qui renferme les molécules amphiphiles, est mis en mouvement sur le substrat de support lors d'une rotation au moyen de l'élément rotatif, ce qui conduit à la propagation de la couche moléculaire.
